# EUROPEAN PATENT APPLICATION

(11) **EP 4 104 835 A1**
(43) Date of publication of application: **21.12.2022**
(21) Application number: 21753994.9
(22) Date of filing: 10.02.2021
(51) Int. Cl.: A61K 31/41, A61K 31/4192, A61P 9/00, A61P 9/04, A61P 25/08, A61P 25/18, A61P 25/28, C07D 249/06, C07D 257/04, C07D 405/12

(54) **TYPE 2 RYANODINE RECEPTOR ACTIVITY INHIBITOR**

(30) Priority: 10.02.2020 JP 2020020656
(71) Applicant: Juntendo Educational Foundation, Tokyo 113-8421 (JP)
(72) Inventor: KUNIHIRO, Nagomi Kurebayashi, Tokyo 113-8421 (JP); MURAYAMA, Takashi, Tokyo 113-8421 (JP); KAGECHIKA, Hiroyuki, Tokyo 113-8510 (JP); MORI, Shuichi, Tokyo 113-8510 (JP); YUASA, Mari, Tokyo 113-8510 (JP); IINUMA, Hiroto, Tokyo 113-8510 (JP)
(74) Representative: Plougmann Vingtoft a/s
(86) International application number: PCT/JP2021/005044
(87) International publication number: WO 2021/162054

(57) **Abstract**

Providing a RyR2 activity inhibitor having an excellent RyR2 activity inhibitory effect.

A type 2 ryanodine receptor activity inhibitor comprising a compound represented by the following formula (α1) or a salt thereof, or a solvate of the compound or a salt thereof, 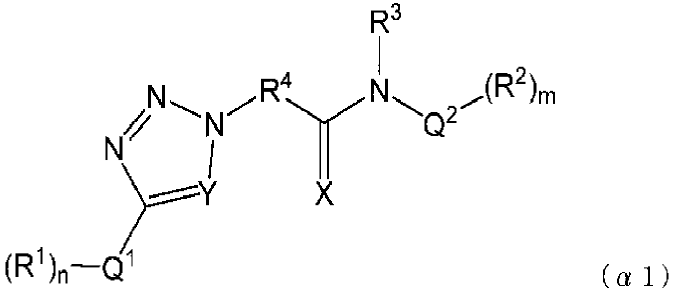 wherein
ring Q¹ and ring Q² each independently represent a condensed ring of a monocyclic heterocycle or monocyclic carbocyclic ring and a benzene ring, or a benzene ring,
R¹ and R² each independently represent a substituted or unsubstituted hydrocarbon group having 1 to 8 carbon atoms, a substituted or unsubstituted alkoxy group having 1 to 8 carbon atoms, a substituted or unsubstituted alkoxycarbonyl group having 2 to 8 carbon atoms, a substituted or unsubstituted alkanoyl group having 2 to 8 carbon atoms, a substituted or unsubstituted alkanoyloxy group having 2 to 8 carbon atoms, a hydroxy group, a cyano group or a halogen atom,
R³ represents a hydrogen atom or a substituted or unsubstituted hydrocarbon group having 1 to 8 carbon atoms,
R⁴ represents a divalent hydrocarbon group having 1 to 8 carbon atoms,
X represents an oxygen atom or a sulfur atom,
Y represents a methine group or a nitrogen atom, and
n and m each independently represent an integer of 0 to 5;
with the proviso that, if n is an integer of 2 to 5, n number of R¹ may be the same or different and two R¹ may join together to form a ring; and if m is an integer of 2 to 5, m number of R² may be the same or different and two R² may join together to form a ring.

## Description

### Technical Field

The present invention relates to a type 2 ryanodine receptor activity inhibitor. More specifically, the present invention relates to type 2 ryanodine receptor activity inhibitor, a medicament, a tetrazole compound and others.

### Background Art

A ryanodine receptor (RyR) is a Ca²⁺ release channel, responsive for calcium ion (Ca²⁺) release from sarcoplasmic reticulum (SR), which is an indispensable step for muscle contraction. RyR opens to release Ca²⁺ into the cytoplasmic matrix upon receiving stimulation of Ca²⁺ from the cytoplasmic matrix (Ca²⁺-induced Ca²⁺ release, CICR). As mentioned above, the working mechanism of RyR is positive feedback. More specifically, a small amount of Ca²⁺ present in the cytoplasmic matrix near the channel induces release of a large amount of Ca²⁺ from SR.

### Citation List

### Patent Literature

Patent Literature 1: WO2019/082940

### Non Patent Literatures

Non Patent Literature 1: J. Electrocardiol. 48 (5), 874-878 (2015)
Non Patent Literature 2: Biochim Biophys Acta Mol Cell Res. 1865: 1687-1697, 2018
Non Patent Literature 3: Acta Neuropathol. 134: 749-767, 2017
Non Patent Literature 4: J Clin Invest. 118: 2230-2245,2008
Non Patent Literature 5: Circ J. 84 (2), 226-234, 2019
Non Patent Literature 6: Heart, Lung and Circulation. 2019 Dec 6.
Non Patent Literature 7: Hum Mutat., 37, 1231-1241, 2016

### Summary of Invention

### Technical Problem

RyRs are classified into RyR1 (type 1 ryanodine receptor), which is primarily expressed in skeletal muscle, RyR2 (type 2 ryanodine receptor), which is primarily expressed in myocardium, and RyR3 (type 3 ryanodine receptor), which is widely expressed in the brain. Up to present, studies by the present inventors have elucidated that a specific quinoline-3-carboxylic acid derivative has an inhibitory effect on activity of RyR1 of these RyRs (Patent Literature 1) but a RyR2 activity inhibitor have not yet been found.

Mutations in RyR2 gene elevate CICR activity abnormally and cause diseases such as catecholaminergic polymorphic ventricular tachycardia, idiopathic ventricular fibrillation, arrhythmogenic right ventricular cardiomyopathy, left ventricular noncompaction, epilepsy and mental retardation. Even in the case of wild-type RyR2, abnormal activation of RyR2 occurs due to e.g., excessive phosphorylation and causes diseases such as chronic heart failure and Alzheimer's disease (for example, e.g., Non Patent Literatures 1 to 6) .

In the circumstances, it has been desired to develop a RyR2 activity inhibitor having an excellent RyR2 activity inhibitory effect.

Accordingly, an object of the present invention is to provide a RyR2 activity inhibitor having an excellent RyR2 activity inhibitory effect.

### Solution to Problem

The present inventors separately cultivated cultured cells expressing a fluorescent endoplasmic reticulum Ca²⁺ indicator and a disease-linked mutant RyR, and cultured cells expressing a fluorescent endoplasmic reticulum Ca²⁺ indicator and a wild type RyR, measured the Ca²⁺ concentration in the endoplasmic reticulum in the presence of a test substance, and compared the Ca²⁺ concentrations in the endoplasmic reticulum of both cultured cells. As a result, they have found that CICR activity inhibitors, more specifically, drugs for preventing or treating diseases associated with abnormally elevated ryanodine receptor activity, can be screened (Non Patent Literature 7) and previously filed a patent application (JP Patent Application No. 2016-113147).

Using the screening method, they screened a wide variety of compounds and further studied. As a result, they have found that a specific compound has an excellent RyR2 activity inhibitory effect and is useful as a drug for preventing or treating diseases associated with abnormally elevated RyR2 activity. Based on the finding, the present invention has been accomplished.

More specifically, the present invention provides the following <1> to <12>.
<1> A type 2 ryanodine receptor activity inhibitor (hereinafter referred to as a type 2 ryanodine receptor activity inhibitor of the present invention or a RyR2 activity inhibitor of the present invention) comprising a compound represented by the following formula (α1) or a salt thereof, or a solvate of the compound or a salt thereof (hereinafter, these will be sometimes collectively referred to as "compound (α1)"), 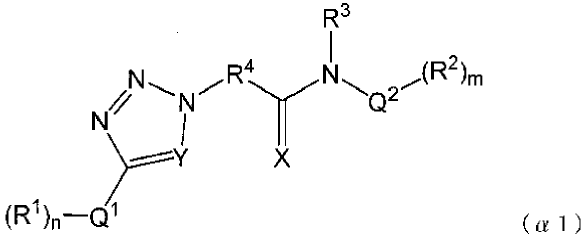 wherein
   ring Q¹ and ring Q² each independently represent a condensed ring of a monocyclic heterocycle or monocyclic carbocyclic ring and a benzene ring, or a benzene ring,
   R¹ and R² each independently represent a substituted or unsubstituted hydrocarbon group having 1 to 8 carbon atoms, a substituted or unsubstituted alkoxy group having 1 to 8 carbon atoms, a substituted or unsubstituted alkoxycarbonyl group having 2 to 8 carbon atoms, a substituted or unsubstituted alkanoyl group having 2 to 8 carbon atoms, a substituted or unsubstituted alkanoyloxy group having 2 to 8 carbon atoms, a hydroxy group, a cyano group or a halogen atom,
   R³ represents a hydrogen atom or a substituted or unsubstituted hydrocarbon group having 1 to 8 carbon atoms,
   R⁴ represents a divalent hydrocarbon group having 1 to 8 carbon atoms,
   X represents an oxygen atom or a sulfur atom,
   Y represents a methine group or a nitrogen atom, and
   n and m each independently represent an integer of 0 to 5;
   with the proviso that, if n is an integer of 2 to 5, n number of R¹ may be the same or different and two R¹ may join together to form a ring; and if m is an integer of 2 to 5, m number of R² may be the same or different and two R² may join together to form a ring.
<2> The type 2 ryanodine receptor activity inhibitor according to <1>, wherein ring Q¹ and ring Q² are each independently a condensed ring of a 4 to 8 membered oxygen-containing monocyclic heterocycle and a benzene ring, or a benzene ring.
<3> The type 2 ryanodine receptor activity inhibitor according to <1> or <2>, wherein R¹ and R² are each independently a substituted or unsubstituted alkyl group having 1 to 8 carbon atoms, a substituted or unsubstituted alkenyl group having 2 to 8 carbon atoms, a substituted or unsubstituted alkoxy group having 1 to 8 carbon atoms, a substituted or unsubstituted alkoxycarbonyl group having 2 to 8 carbon atoms, a hydroxy group, a cyano group or a halogen atom.
<4> The type 2 ryanodine receptor activity inhibitor according to any one of <1> to <3>, wherein R⁴ is an alkanediyl group having 1 to 8 carbon atoms or an alkenediyl group having 2 to 8 carbon atoms.
<5> The type 2 ryanodine receptor activity inhibitor according to any one of <1> to <4>, wherein R³ is a hydrogen atom, a substituted or unsubstituted alkyl group having 1 to 8 carbon atoms or a substituted or unsubstituted alkenyl group having 2 to 8 carbon atoms.
<6> The type 2 ryanodine receptor activity inhibitor according to any one of <1> to <5>, wherein R³ is a methyl group.
<7> The type 2 ryanodine receptor activity inhibitor according to any one of <1> to <6>, wherein n is an integer of 1 to 5.
<8> The type 2 ryanodine receptor activity inhibitor according to any one of <1> to <7>, wherein m is an integer of 1 to 5.
<9> A medicament (hereinafter referred to also as a medicament of the present invention) for preventing or treating a disease associated with abnormally elevated type 2 ryanodine receptor activity, comprising a compound represented by the above formula (α1) or a salt thereof, or a solvate of the compound or a salt thereof.
<10> The medicament according to <9>, wherein the disease associated with abnormally elevated type 2 ryanodine receptor activity is a disease selected from the group consisting of catecholaminergic polymorphic ventricular tachycardia, idiopathic ventricular fibrillation, arrhythmogenic right ventricular cardiomyopathy, left ventricular noncompaction, epilepsy, mental retardation, chronic heart failure and Alzheimer's disease.
<11> A compound represented by the following formula (α2) or a salt thereof, or a solvate of the compound or a salt thereof (hereinafter, these will be sometimes collectively referred to as "compound (α2)"), 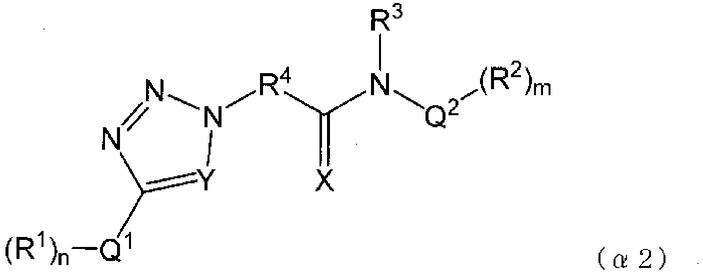 wherein
   ring Q¹ and ring Q² each independently represent a condensed ring of a monocyclic heterocycle or monocyclic carbocyclic ring and a benzene ring, or a benzene ring,
   R¹ and R² each independently represent a substituted or unsubstituted hydrocarbon group having 1 to 8 carbon atoms, a substituted or unsubstituted alkoxy group having 1 to 8 carbon atoms, a substituted or unsubstituted alkoxycarbonyl group having 2 to 8 carbon atoms, a substituted or unsubstituted alkanoyl group having 2 to 8 carbon atoms, a substituted or unsubstituted alkanoyloxy group having 2 to 8 carbon atoms, a hydroxy group, a cyano group or a halogen atom,
   R³ represents a hydrogen atom or a substituted or unsubstituted hydrocarbon group having 1 to 8 carbon atoms,
   R⁴ represents a divalent hydrocarbon group having 1 to 8 carbon atoms,
   X represents an oxygen atom or a sulfur atom,
   Y represents a methine group or a nitrogen atom,
   n and m each independently represent an integer of 1 to 5;
   with the proviso that, if n is an integer of 2 to 5, n number of R¹ may be the same or different and two R¹ may join together to form a ring; and if m is an integer of 2 to 5, m number of R² may be the same or different and two R² may join together to form a ring.
<12> A compound represented by any one of the following formulas (α3) to (α12) or a salt thereof, or a solvate of the compound or a salt thereof (hereinafter, these will be sometimes collectively referred to as "compound (α3) to (α12)". 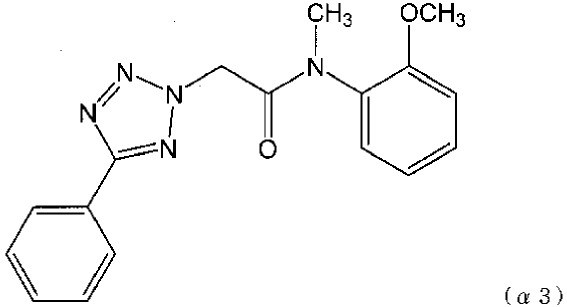 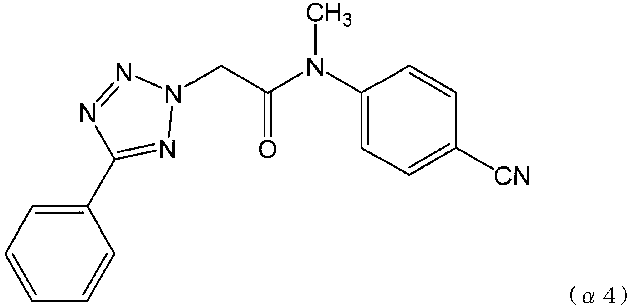 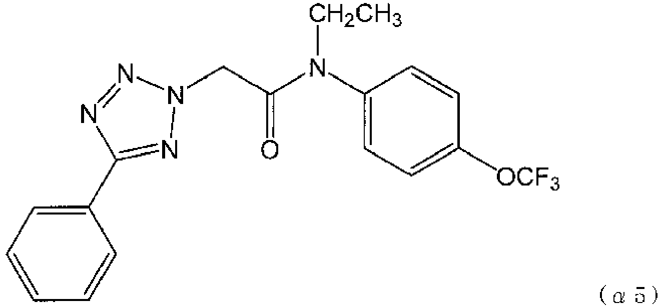 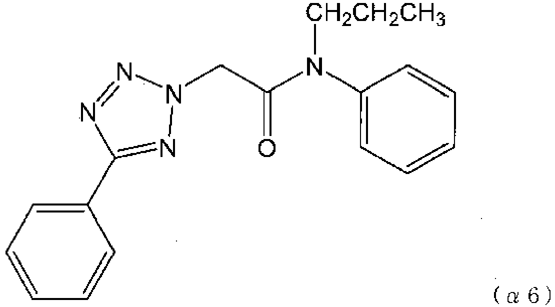 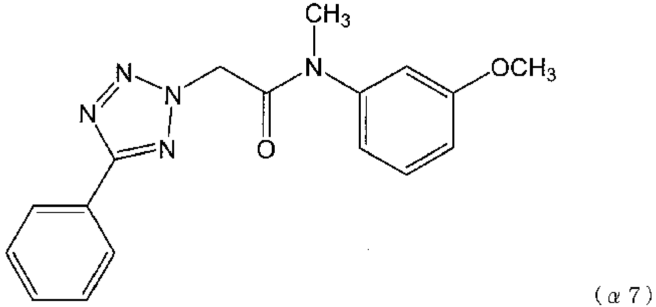 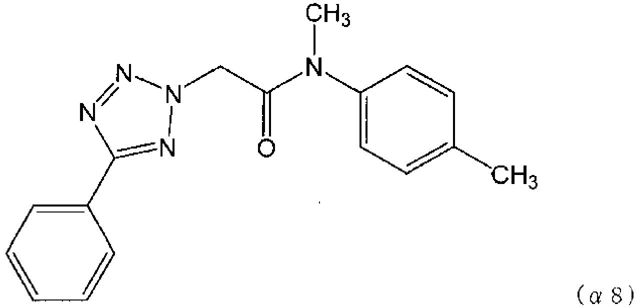 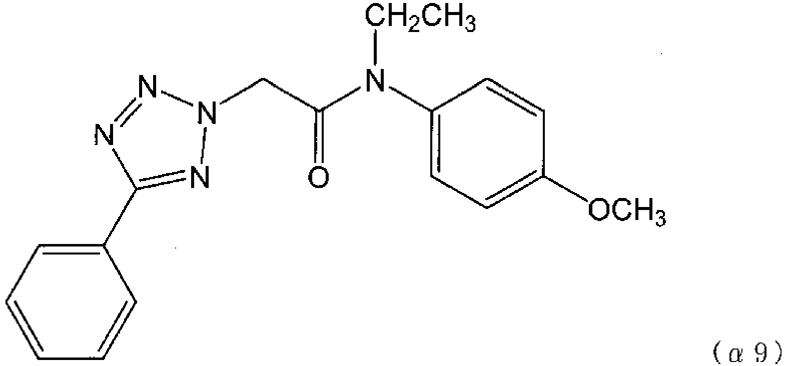 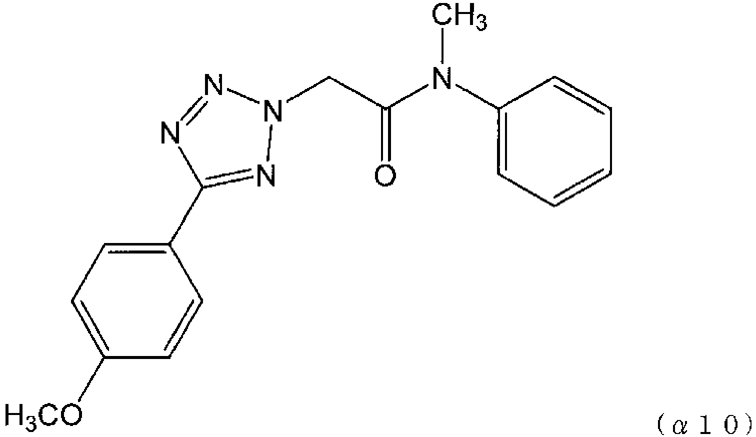 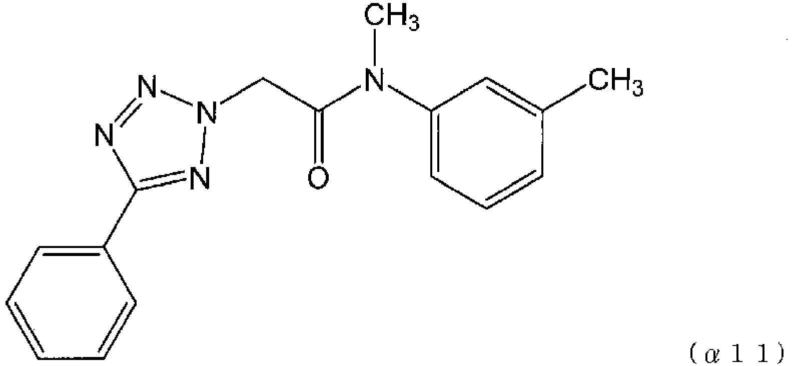 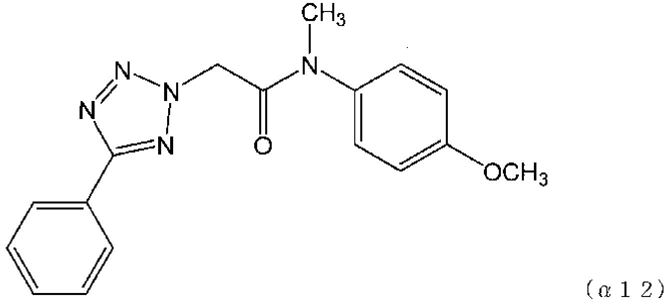
<13> Use of a compound represented by the above formula (α1) or a salt thereof, or a solvate of the compound or a salt thereof, for producing a type 2 ryanodine receptor activity inhibitor.
<14> Use of a compound represented by the above formula (α1) or a salt thereof, or a solvate of the compound or a salt thereof for producing a medicament for preventing or treating a disease associated with abnormally elevated type 2 ryanodine receptor activity.
<15> A compound represented by the above formula (α1) or a salt thereof, or a solvate of the compound or a salt thereof for use in inhibiting a type 2 ryanodine receptor activity.
<16> A compound represented by the above formula (α1) or a salt thereof, or a solvate of the compound or a salt thereof for use in preventing or treating a disease a disease associated with abnormally elevated type 2 ryanodine receptor activity.
<17> Use of a compound represented by the above formula (α1) or a salt thereof, or a solvate of the compound or a salt thereof for inhibiting a type 2 ryanodine receptor activity.
<18> Use of a compound represented by the above formula (α1) or a salt thereof, or a solvate of the compound or a salt thereof for preventing or treating a disease associated with abnormally elevated type 2 ryanodine receptor activity.
<19> A method for inhibiting a type 2 ryanodine receptor activity, comprising a step of administering a compound represented by the above formula (α1) or a salt thereof, or a solvate of the compound or a salt thereof.
<20> A method for preventing or treating a disease associated with abnormally elevated type 2 ryanodine receptor activity, comprising a step of administering a compound represented by the above formula (α1) or a salt thereof, or a solvate of the compound or a salt thereof.

### Advantageous Effects of Invention

A compound represented by formula (α1) or a salt thereof, or a solvate of the compound or a salt thereof has an excellent RyR2 activity inhibitory effect and is useful as a medicament preventing or treating diseases associated with abnormally elevated RyR2 activity.

The compounds (α2) to (α12) of the present invention are novel compounds having an excellent RyR2 activity inhibitory effect.

### Brief Description of Drawings

[Figure 1] An electrocardiogram of a mouse harboring RyR2 mutation before and after administration of compound 57.
[Figure 2] A graph showing the effect of compound 57 on occurrence of arrhythmia of mice harboring RyR2 mutation at a normal time.

### Description of Embodiments

The type 2 ryanodine receptor activity inhibitor and a medicament of the present invention contain a compound represented by the following formula (α1) or a salt thereof, or a solvate of the compound or a salt thereof. It has not been known that a compound represented by formula (α1) or a salt thereof, or a solvate of the compound or a salt thereof has a RyR2 activity inhibitory effect, and is useful as a drug for preventing or treating diseases associated with abnormally elevated RyR2 activity. Note that, in the specification, the "preventing or treating" diseases includes use for not only preventing or treatment diseases but also both preventing and treating diseases.

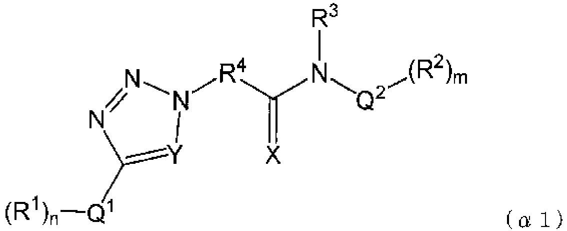

In formula (α1),
ring Q¹ and ring Q² each independently represent a condensed ring of a monocyclic heterocycle or monocyclic carbocyclic ring and a benzene ring, or a benzene ring,
R¹ and R² each independently represent a substituted or non-substituted hydrocarbon group having 1 to 8 carbon atoms, a substituted or non-substituted alkoxy group having 1 to 8 carbon atoms, a substituted or non-substituted alkoxycarbonyl group having 2 to 8 carbon atoms, a substituted or non-substituted alkanoyl group having 2 to 8 carbon atoms, a substituted or non-substituted alkanoyloxy group having 2 to 8 carbon atoms, a hydroxy group, a cyano group, or a halogen atom,
R³ represents a hydrogen atom or a substituted or non-substituted hydrocarbon group having 1 to 8 carbon atoms,
R⁴ represents a divalent hydrocarbon group having 1 to 8 carbon atoms,
X represents an oxygen atom or a sulfur atom,
Y represents a methine group or a nitrogen atom,
n and m each independently represent an integer of 0 to 5;
with the proviso that, if n is an integer of 2 to 5, n number of R¹ may be the same or different and two R¹ may join together to form a ring. If m is an integer of 2 to 5, m number of R² may be the same or different and two R² may join together to form a ring.

First, reference symbols in formulas (α1) and (α2) will be described.

In formulas (α1) and (α2), Y represents a methine group or a nitrogen atom. In view of the RyR2 activity inhibitory effect, Y is preferably a nitrogen atom.

In formulas (α1) and (α2), ring Q¹ and ring Q² each independently represent a condensed ring of a monocyclic heterocycle or monocyclic carbocyclic ring and a benzene ring, or a benzene ring.

The above monocyclic heterocycle is preferably a 4 to 8 membered monocyclic heterocycle, more preferably a 5 to 7 membered monocyclic heterocycle, and particularly preferably, a 5 to 6 membered monocyclic heterocycle. As the monocyclic heterocycle, a monocyclic heterocycle containing one or more heteroatoms selected from the group consisting of an oxygen atom, a nitrogen atom and a sulfur atom is mentioned. An oxygen-containing monocyclic heterocycle is preferable and a 4 to 8 membered oxygen-containing monocyclic heterocycle is more preferable.

Examples of the monocyclic heterocycle include an oxetane ring, dioxolane ring, a dioxane ring, a tetrahydrofuran ring, a tetrahydropyran ring, a tetrahydrothiophene ring, a tetrahydrothiopyran ring, a pyrrolidine ring, a piperidine ring, a piperazine ring and a morpholine ring. Of them, an oxetane ring, a dioxolane ring and a dioxane ring are preferable.

Examples of the condensed ring of a monocyclic heterocycle and a benzene ring include a 7-oxabicyclo[4.2.0]octa-1(6), 2,4-triene ring, a 1,3-benzodioxole ring and a 1,4-benzodioxane ring.

The monocyclic carbon ring is preferably a 4 to 8 membered monocyclic carbocyclic ring, and more preferably, a 5 to 7 membered monocyclic carbocyclic ring. Examples of the condensed ring of a monocyclic carbocyclic ring and a benzene ring include an indane ring and a tetralin ring.

If ring Q¹ is a condensed ring of a monocyclic heterocycle or monocyclic carbocyclic ring and a benzene ring, the binding site of Y and an adjacent carbon atom in formulas (α1) and (α2) is not particularly limited, and a monocyclic heterocycle, a monocyclic carbocyclic ring or a benzene ring contained in a condensed ring may be acceptable and a benzene ring contained in a condensed ring is preferable. If ring Q² is a condensed ring of a monocyclic heterocycle or monocyclic carbocyclic ring and a benzene ring, the binding site of R³ and an adjacent nitrogen atom in formulas (α1) and (α2) is not particularly limited, and a monocyclic heterocycle, a monocyclic carbocyclic ring or a benzene ring contained in a condensed ring is acceptable and a benzene ring contained in a condensed ring is preferable.

Ring Q¹ and ring Q² are, in view of the RyR2 activity inhibitory effect, preferably a condensed ring of a monocyclic heterocycle and a benzene ring, and a benzene ring; and more preferably, a condensed ring of a 4 to 8 membered oxygen-containing monocyclic heterocycle and a benzene ring, and a benzene ring.

Of them, ring Q¹ is, in view of the RyR2 activity inhibitory effect, particularly preferably a benzene ring. Meanwhile, ring Q² is, in view of the RyR2 activity inhibitory effect, particularly preferably a 1,3-benzodioxole ring or a benzene ring.

In formulas (α1) and (α2), R¹ and R² each independently represent a substituted or non-substituted hydrocarbon group having 1 to 8 carbon atoms, a substituted or non-substituted alkoxy group having 1 to 8 carbon atoms, a substituted or non-substituted alkoxycarbonyl group having 2 to 8 carbon atoms, a substituted or non-substituted alkanoyl group having 2 to 8 carbon atoms, a substituted or non-substituted alkanoyloxy group having 2 to 8 carbon atoms, a hydroxy group, a cyano group, or a halogen atom.

In formulas (α1) and (α2), R³ represents a hydrogen atom or a substituted or non-substituted hydrocarbon group having 1 to 8 carbon atoms.

The term "hydrocarbon group" represented by each of R¹, R² and R³, includes an aliphatic hydrocarbon group, an alicyclic hydrocarbon group and an aromatic hydrocarbon group, and is preferably an aliphatic hydrocarbon group.

The aliphatic hydrocarbon group may be linear or branched or saturated or unsaturated. Examples of the aliphatic hydrocarbon group include an alkyl group, an alkenyl group and an alkynyl group. Of them, in view of the RyR2 activity inhibitory effect, an alkyl group and an alkenyl group are preferable, and an alkyl group is more preferable.

The number of carbon atoms of an alkyl group is, in view of the RyR2 activity inhibitory effect, preferably 1 to 8, more preferably 1 to 4, further preferably 1 to 3, and particularly preferably 1. Examples of the alkyl group include a methyl group, an ethyl group, a n-propyl group, an isopropyl group, a n-butyl group, an isobutyl group, a sec-butyl group, a tert-butyl group, a pentyl group and a hexyl group. Of them, in view of the RyR2 activity inhibitory effect, a methyl group, an ethyl group, a n-propyl group and an isopropyl group are preferable, and a methyl group is particularly preferable.

The number of carbon atoms of an alkenyl group and an alkynyl group is preferably 2 to 8, more preferably 2 to 4, and particularly preferably 2 to 3. Examples of the alkenyl group include an ethenyl group, a 1-propenyl group, a 2-propenyl group, a 1-butenyl group, a 2-butenyl group, a 1,3-butadienyl group, a 1-pentenyl group, a 2-pentenyl group and a 1-hexenyl group. Examples of the alkynyl group include an ethynyl group, a 1-propynyl group, a 1-butynyl group, a 1-pentynyl group, a 3-pentynyl group and a 1-hexynyl group.

The "hydrocarbon group" represented by each of R¹, R² and R³ may or may not have a substituent. Examples of the substituent include a halogen atom such as a fluorine atom, a chlorine atom, a bromine atom, and an iodine atom, a hydroxy group and a cyano group. Note that, the position of the substituent and the number of substituents are any position and number. If two or more substituents are present, the substituents may be the same or different. The hydrocarbon group having a substituent is, in view of the RyR2 activity inhibitory effect, preferably a haloalkyl group such as a trifluoromethyl group, a pentafluoroethyl group and a 2,2,2-trifluoroethyl group.

The number of carbon atoms of an "alkoxy group" represented by each of R¹ and R² is, in view of the RyR2 activity inhibitory effect, preferably 1 to 6, more preferably 1 to 3, and particularly preferably 1. The alkoxy group may be linear or branched. Examples of the alkoxy group include a methoxy group, an ethoxy group, a n-propoxy group, an isopropoxy group, a butoxy group, a pentyloxy group and a hexyloxy group.

The "alkoxy group" represented by each of R¹ and R² may or may not have a substituent. Examples of the substituent are the same as those that the aforementioned hydrocarbon groups may have. Note that, the position of the substituent and the number of substituents are any position and number. If two or more substituents are present, the substituents may be the same or different. The alkoxy group having a substituent is, in view of the RyR2 activity inhibitory effect, preferably a haloalkoxy group such as a fluoromethoxy group, a difluoromethoxy group, a trifluoromethoxy group, a pentafluoroethoxy group and a 2,2,2-trifluoroethoxy group.

The number of carbon atoms of an alkoxycarbonyl group, an alkanoyl group and an alkanoyloxy group represented by each of R¹ and R² is, in view of the RyR2 activity inhibitory effect, preferably 2 to 6, and more preferably 2 to 4. The alkoxycarbonyl group, alkanoyl group, and alkanoyloxy group may be linear or branched. Of the alkoxycarbonyl group, alkanoyl group and alkanoyloxy group, an alkoxycarbonyl group is preferable, in view of the RyR2 activity inhibitory effect.

Examples of the alkoxycarbonyl group include a methoxycarbonyl group, an ethoxycarbonyl group, a n-propyloxycarbonyl group, an isopropyloxycarbonyl group, a n-butoxycarbonyl group and a tert-butoxycarbonyl group.

Examples of the alkanoyl group include an acetyl group and a propionyl group.

Examples of the alkanoyloxy group include an acetoxy group and a propanoyloxy group.

Note that, examples of the substituent that the alkoxycarbonyl group, alkanoyl group and alkanoyloxy group may have are the same as those that the aforementioned hydrocarbon groups may have.
Note that, the position of the substituent and the number of substituents are any position and number. If two or more substituents are present, the substituents may be the same or different.

The halogen atom represented by each of R¹ and R² may be a fluorine atom, a chlorine atom, a bromine atom and an iodine atom. Of them, a fluorine atom is preferable.

R¹ and R² are, in view of the RyR2 activity inhibitory effect, preferably a substituted or non-substituted alkyl group having 1 to 8 carbon atoms, a substituted or non-substituted alkenyl group having 2 to 8 carbon atoms, a substituted or non-substituted alkoxy group having 1 to 8 carbon atoms, a substituted or non-substituted alkoxycarbonyl group having 2 to 8 carbon atoms, a hydroxy group, a cyano group, or a halogen atom; more preferably a substituted or non-substituted alkyl group having 1 to 8 carbon atoms, a substituted or non-substituted alkenyl group having 2 to 8 carbon atoms, a substituted or non-substituted alkoxy group having 1 to 8 carbon atoms, a substituted or non-substituted alkoxycarbonyl group having 2 to 8 carbon atoms, a cyano group, or a halogen atom; further preferably a substituted or non-substituted alkyl group having 1 to 8 carbon atoms, a substituted or non-substituted alkoxy group having 1 to 8 carbon atoms, a substituted or non-substituted alkoxycarbonyl group having 2 to 8 carbon atoms, a cyano group or a halogen atom; further preferably a substituted or non-substituted alkoxy group having 1 to 8 carbon atoms or a halogen atom; and particularly preferablyf a halogen atom.

Note that, the substitution position of R¹ may be on ring Q¹. In view of the RyR2 activity inhibitory effect, if ring Q¹ is a benzene ring and n is an integer of 1 to 5, it is preferable that the substitution position of R¹ is at least the 3-position. A combination of the integer n and the substitution position of R¹ is, in view of the RyR2 activity inhibitory effect, preferably a combination of n = 1 and a substitution at the 3-position, and a combination of n = 2 or 3 and substitutions at a position selected from the 4-position and 5-position, and at the 3-position. Note that, the substitution position of R² may be on ring Q². In view of the RyR2 activity inhibitory effect, if ring Q² is a benzene ring and m is an integer of 1 to 5, it is preferable that the substitution position of R² is at least the 4-position. A combination of the integer m and the substitution position of R² is, in view of the RyR2 activity inhibitory effect, preferably a combination of m = 1 and a substitution at the 4-position and a combination of m = 2 and substitutions at the 3-position and 4-position.

R³ represents, in view of the RyR2 activity inhibitory effect, preferably a hydrogen atom, a substituted or non-substituted alkyl group having 1 to 8 carbon atoms, or a substituted or non-substituted alkenyl group having 2 to 8 carbon atoms; more preferably a hydrogen atom, or a substituted or non-substituted alkyl group having 1 to 8 carbon atoms; further preferably a substituted or non-substituted alkyl group having 1 to 8 carbon atoms; further preferably a substituted or non-substituted alkyl group having 1 to 4 carbon atoms; and particularly preferably a methyl group.

In formulas (α1) and (α2), R⁴ represents a divalent hydrocarbon group having 1 to 8 carbon atoms.

The concept term of a divalent hydrocarbon group represented by R⁴ includes a divalent aliphatic hydrocarbon group, a divalent alicyclic hydrocarbon group, and a divalent aromatic hydrocarbon group. Of them, a divalent aliphatic hydrocarbon group is preferable.

The divalent aliphatic hydrocarbon group may be linear or branched and saturated or unsaturated. Examples of the divalent aliphatic hydrocarbon group include an alkanediyl group, an alkenediyl group and an alkynediyl group. Of them, in view of the RyR2 activity inhibitory effect, an alkanediyl group and alkenediyl group are preferable, and an alkanediyl group is more preferable.

The number of carbon atoms of an alkanediyl group is, in view of the RyR2 activity inhibitory effect, preferably 1 to 8, more preferably 1 to 4, further preferably 1 to 3, and particularly preferably 1. Examples of the alkanediyl group include a methane-1,1-diyl group, an ethane-1,1-diyl group, an ethane-1,2-diyl group, a propane-1,1-diyl group, a propane-1,2-diyl group, a propane-1,3-diyl group, a propane-2,2-diyl group, a butane-1,1-diyl group, a butane-1,2-diyl group, a butane-1,3-diyl group, a butane-1,4-diyl group, a pentane-1,4-diyl group, a pentane-1,5-diyl group, a hexane-1,2-diyl group, a hexane-1,5-diyl group, a hexane-1,6-diyl group, a heptane-1,7-diyl group and an octane-1,8-diyl group. Of them, in view of the RyR2 activity inhibitory effect, a methane-1,1-diyl group is preferable.

The number of carbon atoms of an alkenediyl group and an alkynediyl group is preferably 2 to 8, more preferably 2 to 4, and particularly preferably 2 or 3. Examples of the alkenediyl group include ethylene-1,1-diyl group and an ethylene-1,2-diyl group. Examples of the alkynediyl group include an acetylene-1,2-diyl group.

In formula (α1), n and m each independently represent an integer of 0 to 5. In view of the RyR2 activity inhibitory effect, n and m may be an integer of preferably 1 to 5, more preferably 1 to 3, and particularly preferably 1 or 2.

Similarly, in formula (α2), n and m may be, in view of the RyR2 activity inhibitory effect, an integer of preferably 1 to 3, and more preferably, 1 or 2.

Compounds (α1) to (α12) include salts of the compounds represented by formulas (α1) to (α12). Examples of the salts include alkali metal salts such as a sodium salt and a potassium salt; salts with a group-2 metal element such as a calcium salt and a magnesium salt; salts with a group-13 metal element such as an aluminum salt; ammonium salts; and organic amine salts such as a phenethylamine salt. In compounds (α1) to (α12), solvates thereof include a hydrate and solvates of alcohols.

Compounds (α1) to (α12) may sometimes have crystal polymorphic forms. Compounds (α1) to (α12) may have a single crystal form or a mixture of a plurality of crystal forms. Alternatively, they may have amorphous form.

Of the compounds represented by formula (α1), compound (α2), compound (α3) to (α12) are novel compounds. Compound (α2) is a compound represented by formula (α1) where n and m each represent an integer of 1 to 5. A compound represented by formula (α3) is compound 39 later described in Examples; a compound represented by formula (α4) is compound 44 later described in Examples; a compound represented by formula (α5) is compound 61 later described in Examples; a compound represented by formula (α6) is compound 66 later described in Examples; a compound represented by formula (α7) is compound 40 later described in Examples; a compound represented by formula (α8) is compound 38 later described in Examples; a compound represented by formula (α9) is compound 59 later described in Examples; a compound represented by formula (α10) is compound 47 later described in Examples; a compound represented by formula (α11) is compound 37 later described in Examples; and a compound represented by formula (α12) is compound 41 later described in Examples;

Now, a method for producing compound (α1) will be described. Compound (α1) can be produced from a nitrile compound (CP1) and bromocarboxylic acid (CP6) used as starting materials by appropriate combination of methods commonly known and described, for example, in N. T. Pokhodylo, R. D. Savka, V. S. Matiichuk, and N. D. Obushak, A Study of Alkylation Regioselectivity of 5-Substituted Tetrazoles with Chloroacetamides; and Russian Journal of General Chemistry, 80, 836-841 (2010).

As shown in the following synthetic pathway, for example, a compound (α1) represented by formula (α1) wherein Y is a nitrogen atom, X is an oxygen atom; and R³ is a hydrogen atom, may be produced by
<Step SA1-1> reacting sodium azide with a nitrile compound (CP1) in the presence of an amine salt to obtain a tetrazole derivative (CP2),
<Step SA1-2> amidating an aniline derivative (CP3) and bromocarboxylic acid chloride by use of e.g., a base, to obtain an amide derivative (CP4), and
<Step SA2> reacting the tetrazole derivative (CP2) and the amide derivative (CP4) in the presence of e.g., a strong base.

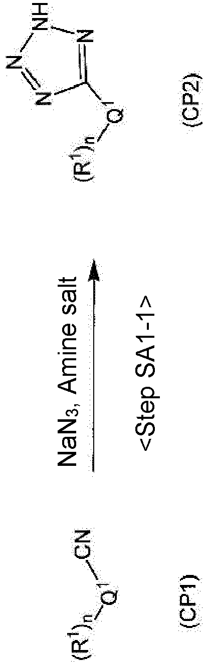

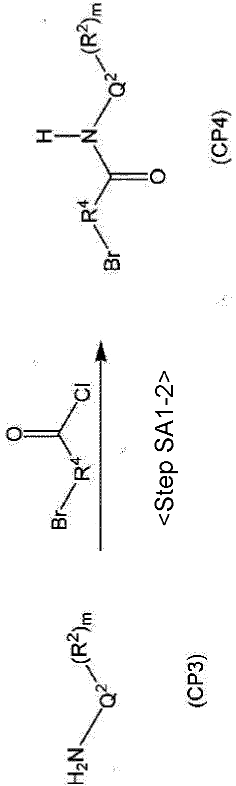

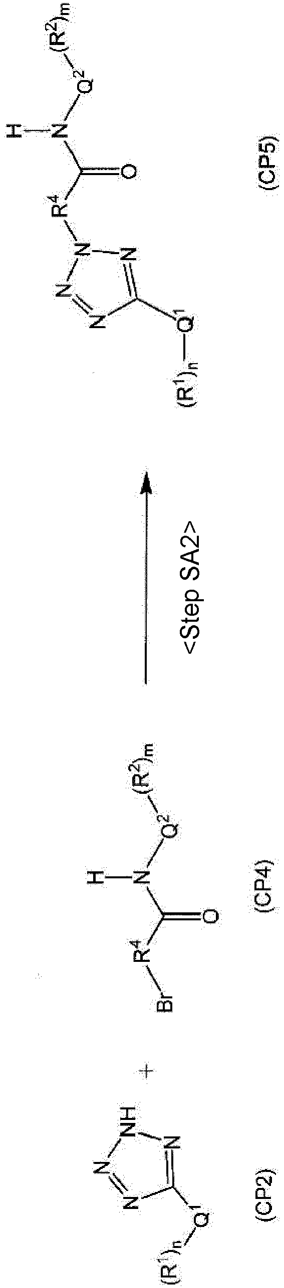

A compound (α1) represented by formula (α1) wherein Y is a methine group; X is an oxygen atom; and R³ is a hydrogen atom, may be produced by
<Step SB1> reacting bromocarboxylic acid (CP6) and sodium azide in water,
<Step SB2> converting azidocarboxylic acid (CP7) obtained above into an acid chloride by use of oxalyl chloride and then amidating the acid chloride and an aniline derivative (CP8) by use of e.g., a base, to obtain an azide group-containing amide derivative (CP9), and
<Step SB3> reacting the azide group-containing amide derivative (CP9) and an ethynyl compound (CP10) in the presence of a metal catalyst such as copper sulfate pentahydrate and sodium ascorbate.

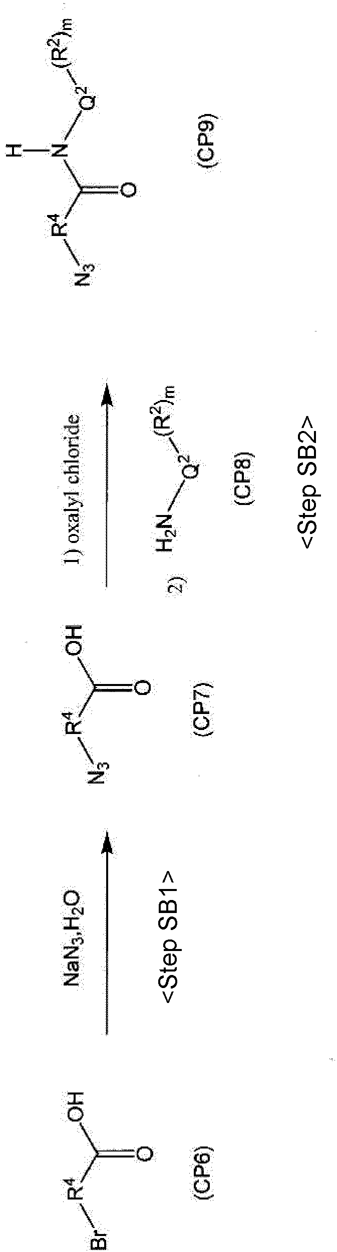

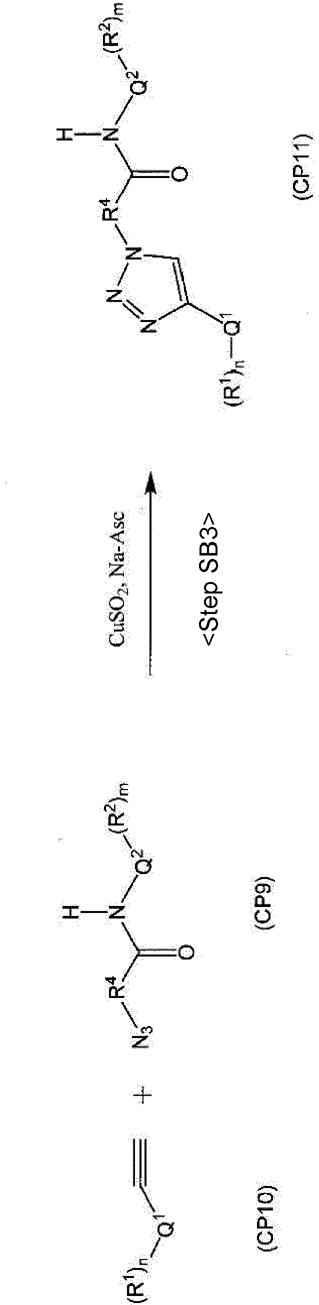

With the compounds (CP5) and (CP11) obtained above, an iodinated hydrocarbon is reacted in the presence of a base such as cesium carbonate to obtain a compound represented by formula (α1) wherein R³ is a hydrocarbon group. With the compound (CP5), the compound (CP11), the compound obtained by reacting an iodinated hydrocarbon to these compounds, a sulfating agent such as Lawesson reagent is reacted to obtain a compound represented by formula (α1) wherein X is a sulfur atom.

A compound represented by the above formula (α1) or a salt thereof, or a solvate of the compound or a salt thereof, which can be produced as described above, has an excellent RyR2 activity inhibitory effect and is useful as a drug for preventing or treating diseases associated with abnormally elevated RyR2 activity.

The diseases associated with abnormally elevated RyR2 activity may be diseases caused by a RyR2 mutation (more specifically, activity-enhancing mutation) or diseases associated with abnormally elevated wild-type RyR2 activity. Examples of the diseases include arrhythmia, heart failure, cardiomyopathy, neurological disorder and Alzheimer's disease. Examples of the diseases caused by RyR2 activity-enhancing mutation include catecholaminergic polymorphic ventricular tachycardia, idiopathic ventricular fibrillation, arrhythmogenic right ventricular cardiomyopathy, left ventricular noncompaction, epilepsy and mental retardation. Examples of the disease associated with abnormally elevated wild-type RyR2 activity include chronic heart failure associated with sympathetic tone in wild-type RyR2 and Alzheimer's disease. The compound (α1) of the present invention is particularly useful as a drug for preventing or treating a disease selected from the group consisting of catecholaminergic polymorphic ventricular tachycardia, idiopathic ventricular fibrillation, arrhythmogenic right ventricular cardiomyopathy, left ventricular noncompaction, epilepsy, mental retardation, chronic heart failure and Alzheimer's disease.

Accordingly, the compound (α1) of the present invention can serve as a RyR2 activity inhibitor and a medicament for preventing or treating a disease associated with abnormally elevated RyR2 activity; and can be used for inhibiting RyR2 activity and preventing or treating a disease associated with abnormally elevated RyR2 activity and for producing a RyR2 activity inhibitor and a medicament for preventing or treating a disease associated with abnormally elevated RyR2 activity. Note that, the term "use" may refer to administration to or ingestion by humans or non-human animals.

The RyR2 activity inhibitor and a medicament of the present invention may be applied by either a parenteral means such as injection, transrectal and topical administration or oral means. The compound (α1) of the present invention may be used in combination with a pharmaceutically acceptable carrier and formulated into a pharmaceutical composition. Examples of the pharmaceutically acceptable carrier include those commonly known in the technical field, such as an excipient, a binder, a buffer, a thickener, a stabilizer, an emulsifier, a dispersant, a suspending agent and a preservative. The pharmaceutical composition can be prepared into drug preparations in accordance with a method commonly used in the technical field.

Examples of the drug preparations for oral administration include tablets (including sugar-coated tablets and film coated tablets), pills, granules, powders, capsules (including soft capsules), syrups, emulsions and suspensions.

The drug preparation for oral administration can be produced by using additives commonly employed in the pharmaceutical field in accordance with a method commonly known. Examples of the additives include excipients such as lactose, mannitol, anhydrous calcium hydrogen phosphate; binders such as hydroxypropyl cellulose, methyl cellulose and polyvinylpyrrolidone; disintegrants such as starch and carboxymethyl cellulose; and lubricants such as magnesium stearate and talc.

Examples of the drug preparation for parenteral administration include injections, rectal administration preparations and topical administration preparations. Of them, an injection is preferable.

Examples of the injections include an aseptic solution and suspension containing the compound (α1) of the present invention. An injection can be produced, for example, by dissolving or suspending the compound (α1) of the present invention in water for injection according to the Japanese Pharmacopoeia. An injection may contain, if necessary, a tonicity agent such as sodium chloride; a buffer such as sodium dihydrogen phosphate and sodium monohydrogen phosphate; and a dissolution aid. Also, an injection may be provided as a powder or lyophilized preparation, which is prepared into the injection when used by dissolving the preparation. In this case, a preparation can be produced using an excipient such as mannitol and lactose, in accordance with a common method.

Examples of the rectal administration preparations include a suppository. The suppository can be prepared, for example, by dissolving or suspending the compound (α1) of the present invention in a base such as cocoa butter and macrogol, and thereafter, pouring the solution or suspension in a template and molding it. Alternatively, a rectal administration preparation can be produced by placing a solution or a cream in a container for injection.

Examples of dosage forms of topical administration preparations include a liquid, an eye drop, a cream, an ointment, a gel, a spray and a powder.

The liquid can be produced by adding the compound (α1) of the present invention in water and, if necessary, adding, e.g., a stabilizer, a dissolving agent, a thickener, a dispersant and a suspending agent.

The eye drop can be produced by adding, for example, the compound (α1) of the present invention, a buffer, a pH regulator, a tonicity agent and a preservative.

The cream and ointment can be produced by using, for example, the compound (α1) of the present invention together with an aqueous or oily base (for example, water, liquid paraffin, vegetable oil (e.g., peanut oil, castor oil), macrogol).

The gel can be produced by using, for example, the compound (α1) of the present invention together with, e.g., gelatin, pectin, carrageenan, agar, tragacanth, an alginate, cellulose ether (e.g., methyl cellulose, sodium carboxymethyl cellulose), a pectin derivative, polyacrylate, polymethacrylate, polyvinyl alcohol and polyvinylpyrrolidone.

The spray can be prepared by dissolving or suspending, for example, the compound (α1) of the present invention in, e.g., water, and charging a spray container with the solution or suspension.

In the case of a powder, the compound (α1) of the present invention can be directly used or may be blended with an appropriate excipient.

The dose or injection amount of the RyR2 activity inhibitor or medicament of the present invention is determined in consideration of a target disease and symptom, and the age, body weight and sex of the subject to be administered. In the case of oral administration, the dose of the compound (α1) of the present invention is usually 0.01 to 100 mg, preferably 0.01 to 30 mg, and further preferably 0.1 to 10 mg per adult (body weight: about 60 kg) per day. The dose can be administered or ingested once or divided into 2 to 4 portions and separately administered or ingested. In the case of intravenous administration, the dose of the compound (α1) of the present invention is usually 0.03 to 3000 µg, preferably 0.03 to 300 µg and more preferably 0.03 to 30 µg per adult/body weight (1 kg)/day. The dose is administered once a day or divided into portions and separately administered.

### Examples

Now, the present invention will be described in detail by way of Examples but the invention is not limited by Examples. Note that, ¹H NMR spectrum was measured by use of AVANCE 400 or AVANCE 500 spectrometer manufactured by Bruker.

### (Synthesis Example 1: Synthesis of Compound 1)

Compound 1 was synthesized in accordance with the following synthetic pathway.

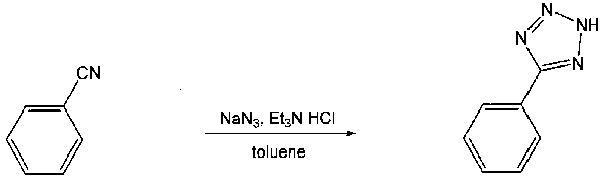

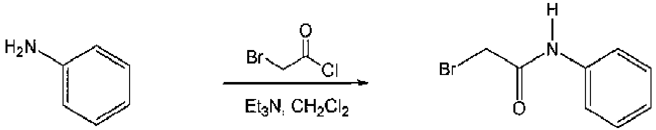

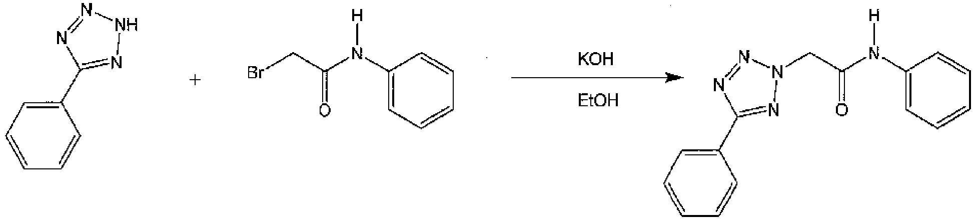

### (1-1) Synthesis of Tetrazole Derivative

In a 300 mL eggplant-shaped flask, benzonitrile (3 mmol) was dissolved in toluene (80 mL). To the solution, sodium azide (586 mg, 9 mmol), and triethylamine hydrochloride (1.24 g, 9 mmol) were added. The reaction solution was stirred at 100°C for 24 hours. After the temperature of the reaction solution was returned to room temperature, the reaction solution was added to concentrated hydrochloric acid/ice. The precipitate was collected by suction filtration. The resultant solid substance was washed with water to obtain a tetrazole derivative as a white solid substance (yield: 90%).

### (1-2) Synthesis of Amide Derivative

In a 100 mL eggplant-shaped flask, bromoacethyl chloride (4.72 g, 13.4 mmol) was dissolved in dichloromethane. The solution was stirred at 0°C. To the solution, aniline (10.7 mmol) dissolved in dichloromethane and triethylamine (14.4 mmol) were added dropwise. The reaction solution was further stirred for one hour. After the temperature of the reaction solution was returned to room temperature, the precipitated solid substance was subjected to suction filtration. The obtained solution was separated into layers by use of water and dichloromethane. After the organic layer was dried over anhydrous sodium sulfate, the solvent was distilled away. The residue was purified by silica gel column chromatography to obtain an amide derivative as a brown solid substance (yield: 78%).

### (1-3) Synthesis of Compound 1

In a 50 mL eggplant-shaped flask, the tetrazole derivative (2.5 mmol) obtained above and potassium hydroxide were dissolved in ethanol (20 mL). To the solution, the amide derivative (2.5 mmol) obtained above was added. The reaction solution was heated to reflux for 16 hours. After the temperature of the reaction solution was returned to room temperature, water (50 mL) was added to the reaction solution. The precipitated solid substance was collected by suction filtration and recrystallized from ethanol to obtain compound 1 as a colorless needle-like crystal (yield: 60%).

The ¹H NMR spectrum of compound 1 obtained is shown below.

### Compound 1

¹H NMR (500 MHz, DMSO-d₆): δ = 10.63 (br s, 1H), 8.08 (dd, J = 7.8, 2.1 Hz, 2H), 7.59-7.56 (m, 5H), 7.34 (t, J = 7.4 Hz, 2H), 7.10 (t, J = 7.4 Hz, 1H), 5.76 (s, 2H).

### (Synthesis Example 2 to 12: Synthesis of Compounds 2 to 12)

Compounds 2 to 12 were synthesized in the same operation as in Synthesis Example 1 except that substituted anilines shown in Tables 1 and 2 were used in place of the aniline used for synthesis of an amide derivative.

**[Table 1]**

| Synthesis Example | Substituted aniline |
|---|---|
| 2 | 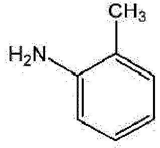 |
| 3 | 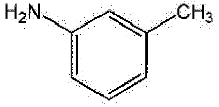 |
| 4 | 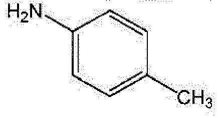 |
| 5 | 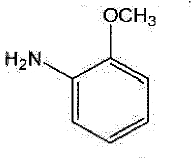 |
| 6 | 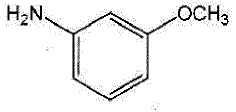 |

**[Table 2]**

| Synthesis Example | Substituted aniline |
|---|---|
| 7 | 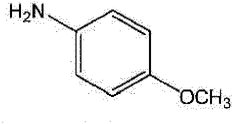 |
| 8 | 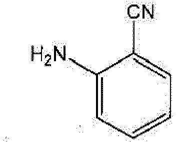 |
| 9 | 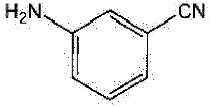 |
| 10 | 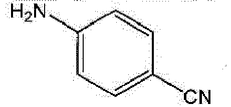 |
| 11 | 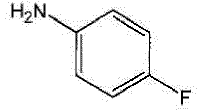 |
| 12 | 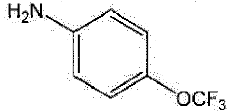 |

The ¹H NMR spectra of the resultant compounds 2 to 12 are shown below.

### Compound 2

¹H NMR (500 MHz, DMSO-d₆): δ = 9.95 (br s, 1H), 8.08 (dd, J = 8.1, 1.5 Hz, 2H), 7.59-7.53 (m, 3H), 7.42 (d, J = 7.0 Hz, 1H), 7.23 (d, J = 7.2 Hz, 1H), 7.17 (t, J = 7.2 Hz, 1H), 7.17 (td, J = 7.4, 1.3 Hz, 1H), 5.80 (s, 2H), 2.25 (s, 3H).

### Compound 3

¹H NMR (500 MHz, DMSO-d₆): δ = 10.55 (br s, 1H), 8.08 (dd, J = 8.0, 2.0 Hz, 2H), 7.59-7.53 (m, 3H), 7.42 (s, 1H), 7.36 (d, J = 8.2 Hz, 2H), 7.21 (t, J = 7.8 Hz, 3H), 6.92 (d, J = 7.5 Hz, 3H), 5.74 (s, 2H), 2.27 (s, 3H).

### Compound 4

¹H NMR (500 MHz, DMSO-d₆): δ = 10.54 (br s, 1H), 8.08 (dd, J = 8.0, 2.0 Hz, 2H), 7.59-7.54 (m, 3H), 7.46 (d, J = 8.4 Hz, 2H), 7.14 (d, J = 7.3 Hz, 2H), 5.73 (s, 2H), 2.25 (s, 3H).

### Compound 5

¹H NMR (500 MHz, DMSO-d₆): δ = 9.92 (br s, 1H), 8.07 (dd, J = 8.0, 1.6 Hz, 2H), 7.92 (d, J = 8.0 Hz, 1H), 7.59-7.53 (m, 3H), 7.13-7.08 (m, 2H), 6.90 (td, J = 7.9, 1.7 Hz, 1H), 5.86 (s, 2H), 3.88 (s, 3H).

### Compound 6

¹H NMR (500 MHz, DMSO-d₆): δ = 10.62 (br s, 1H), 8.08 (dd, J = 8.0, 1.9 Hz, 2H), 7.59-7.55 (m, 3H), 7.27 (t, J = 2.2 Hz, 1H), 7.24 (t, J = 8.2 Hz, 1H), 7.10 (dd, J = 8.0, 1.1 Hz, 1H), 6.68 (dd, J = 7.9, 1.9 Hz, 1H), 5.75 (s, 2H), 3.71 (s, 3H).

### Compound 7

¹H NMR (500 MHz, DMSO-d₆): δ = 10.49 (br s, 1H), 8.08 (dd, J = 8.0, 1.9 Hz, 2H), 7.58-7.54 (m, 3H), 7.49 (d, J = 9.1 Hz, 2H), 6.91 (d, J = 9.1 Hz, 1H), 5.71 (s, 2H), 3.72 (s, 3H).

### Compound 8

¹H NMR (500 MHz, DMSO-d₆): δ = 0.85 (br s, 1H), 8.08 (dd, J = 7.9, 1.9 Hz, 2H), 7.86 (dd, J = 7.8, 1.0 Hz, 1H), 7.73-7.67 (m, 2H), 7.59-7.55 (m, 3H), 7.39 (td, J = 7.2 Hz, 2H), 5.87 (s, 2H).

### Compound 9

¹H NMR (500 MHz, DMSO-d₆): δ = 10.99 (br s, 1H), 8.09-8.06 (m, 2H), 8.04-8.03 (m, 1H), 7.82-7.80 (m, 1H), 7.59-7.55 (m, 5H), 5.81 (s, 2H).

### Compound 10

¹H NMR (500 MHz, DMSO-d₆): δ = 11.13 (br s, 1H), 8.09-8.06 (m, 2H), 7.82 (d, J = 9.1 Hz, 2H), 7.77 (d, J = 8.8 Hz, 2H), 7.59-7.55 (m, 3H), 5.83 (s, 2H).

### Compound 11

¹H NMR (500 MHz, MeOD): δ = 8.13 (dd, J = 7.7, 1.9 Hz, 2H), 7.59 (dd, J = 7.0, 2.0 Hz, 2H), 7.53-7.50 (m, 3H), 7.07 (t, J = 8.8 Hz, 2H), 5.66 (s, 2H) ; ¹³C NMR (125 MHz, MeOD) δ 163.5 (d, J = 166 Hz), 158.3 (d, J = 239 Hz), 134.7, 130.7, 129.3, 126.8, 126.4, 121.2, 121.1, 115.6, 115.5, 55.3.

### Compound 12

¹H NMR (500 MHz, DMSO-d₆): δ = 11.11 (br s, 1H), 8.08 (d, J = 7.8 Hz, 2H), 7.72 (d, J = 8.7 Hz, 2H), 7.58-7.55 (m, 3 H), 7.35 (d, J = 8.7 Hz, 2H), 5.81 (s, 2H), 3.82 (s, 3H), 3.71 (s, 3H); ¹³C NMR (125 MHz, DMSO-d₆) δ 164.2, 163.2, 144.0, 137.5, 130.7, 129.3, 126.8, 126.4, 121.8, 121.1, 120.8, 119.0, 55.3.

### (Synthesis Examples 13 to 15: Synthesis of Compounds 13 to 15)

Compounds 13 to 15 were synthesized in the same operation as in Synthesis Example 1 except that substituted benzonitriles shown in Table 3 were used in place of the benzonitrile used in synthesis of a tetrazole derivative.

**[Table 3]**

| Synthesis Example | Substituted benzonitrile |
|---|---|
| 13 | 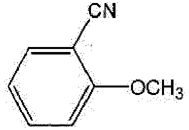 |
| 14 | 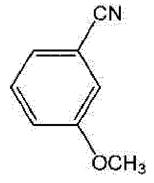 |
| 15 | 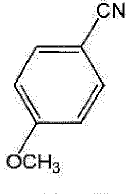 |

The ¹H NMR spectra of the compounds 13 to 15 obtained are shown below.

### Compound 13

¹H NMR (400 MHz, DMSO-d₆): δ = 10.61 (br s, 1H), 7.83 (dd, J = 7.9, 1.8 Hz, 1H), 7.58 (d, J = 8.5 Hz, 2H), 7.52 (dt, J = 8.1, 1.8 Hz, 1H), 7.34 (t, J = 7.9 Hz, 2H), 7.22 (d, J = 8.1 Hz, 1H), 7.12 (tt, J =7.5, 1.4 Hz, 2H), 5.73 (s, 2H), 3.84 (s, 3H),

### Compound 14

¹H NMR (400 MHz, DMSO-d₆): δ = 10.66 (br s, 1H), 7.66 (d, J = 7.7 Hz, 1H), 7.59-7.56 (m, 3H), 7.48 (t, J = 8.1 Hz, 1H), 7.34 (t, J = 8.3 Hz, 2H), 7.13- 7.08 (m, 2H), 5.76 (s, 2H), 3.84 (s, 3H),

### Compound 15

¹H NMR (400 MHz, DMSO-d₆): δ = 10.66 (br s, 1H), 8.01 (d, J = 8.9 Hz, 2H), 7.58 (d, J = 8.4 Hz, 2H), 7.34 (t, J = 7.9 Hz, 2H), 7.13-7.08 (m, 3H), 5.72 (s, 2H), 3.82 (s, 3H)

### (Synthesis Examples 16 to 34: Synthesis of Compounds 16 to 34)

Compounds 16 to 34 were synthesized in the same operation as in Synthesis Example 1 except that substituted benzonitriles shown in Tables 4 to 7 were used in place of the benzonitrile used in synthesis of a tetrazole derivative, and that substituted anilines shown in Tables 4 to 7 were used in place of the aniline used in synthesis of an amide derivative.

**[Table 4]**

| Synthesis Example | Substituted benzonitrile | Substituted aniline |
|---|---|---|
| 16 | The same compound as in Synthesis Example 14 | The same compound as in Synthesis Example 7 |
| 17 | The same compound as in Synthesis Example 14 | The same compound as in Synthesis Example 11 |
| 18 | 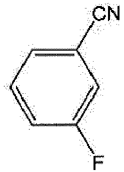 | The same compound as in Synthesis Example 7 |
| 19 | The same compound as in Synthesis Example 18 | The same compound as in Synthesis Example 11 |
| 20 | 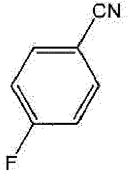 | The same compound as in Synthesis Example 7 |

**[Table 5]**

| Synthesis Example | Substituted benzonitrile | Substituted aniline |
|---|---|---|
| 21 | The same compound as in Synthesis Example 20 | The same compound as in Synthesis Example 12 |
| 22 | The same compound as in Synthesis Example 14 | 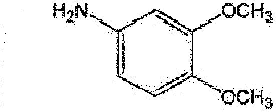 |
| 23 | The same compound as in Synthesis Example 14 | 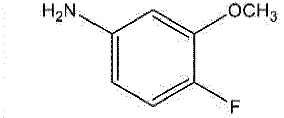 |
| 24 | The same compound as in Synthesis Example 14 | 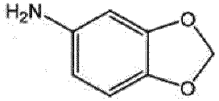 |
| 25 | 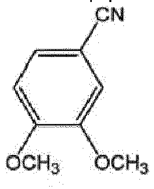 | The same compound as in Synthesis Example 11 |

**[Table 6]**

| Synthesis Example | Substituted benzonitrile | Substituted aniline |
|---|---|---|
| 26 | 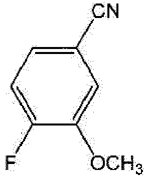 | The same compound as in Synthesis Example 11 |
| 27 | 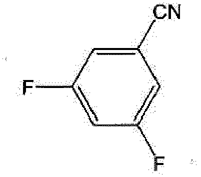 | The same compound as in Synthesis Example 11 |
| 28 | The same compound as in Synthesis Example 15 | The same compound as in Synthesis Example 7 |
| 29 | 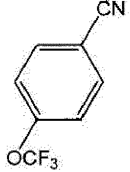 | The same compound as in Synthesis Example 11 |
| 30 | The same compound as in Synthesis Example 29 | The same compound as in Synthesis Example 7 |

**[Table 7]**

| Synthesis Example | Substituted benzonitrile | Substituted aniline |
|---|---|---|
| 31 | The same compound as in Synthesis Example 29 | The same compound as in Synthesis Example 12 |
| 32 | 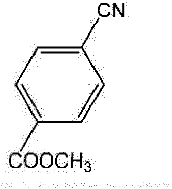 | The same compound as in Synthesis Example 7 |
| 33 | The same compound as in Synthesis Example 32 | The same compound as in Synthesis Example 11 |
| 34 | The same compound as in Synthesis Example 32 | The same compound as in Synthesis Example 12 |

The ¹H NMR spectra of the compounds 16 to 34 obtained are shown below.

### Compound 16

¹H NMR (500 MHz, DMSO-d₆): δ = 10.50 (br s, 1H), 7.65 (dt, J = 7.5, 1.1 Hz, 1H), 7.56-7.55 (m, 1H), 7.50-7.46 (m, 3H), 7.11 (ddd, J = 8.4, 1.5, 0.9, Hz, 1H), 6.90 (d, J = 9.1 Hz, 2H), 5.69 (s, 2H), 3.82 (s, 3H), 3.70 (s, 3 H).

### Compound 17

¹H NMR (500 MHz, DMSO-d₆): δ = 10.70 (br s, 1H), 7.65 (dt, J = 7.8, 1.1 Hz, 1H), 7.59-7.55 (m, 2H), 7.48 (t, J = 8.0 Hz, 2H), 7.17 (t, J = 9.0 Hz, 2H), 7.11 (ddd, J = 8.4, 2.7, 0.9 Hz, 1H), 5.73 (s, 2H), 3.82 (s, 3H).

### Compound 18

¹H NMR (500 MHz, DMSO-d₆): δ = 10.50 (br s, 1H), 7.93 (dt, J = 8.1, 1.3 Hz, 1H), 7.82 (ddd, J = 9.7, 2.6, 1.5 Hz, 1H), 7.63 (dd, J = 10.1, 4.0 Hz, 2H), 7.49 (d, J = 9.1 Hz, 2H), 7.41 (ddd, J = 8.8, 2.8, 0.9 Hz, 1H), 6.91 (d, J = 9.0 Hz, 2H), 5.73 (s, 2H), 3.72 (s, 3H).

### Compound 19

¹H NMR (500 MHz, DMSO-d₆): δ = 10.69 (br s, 1H), 7.93 (dt, J = 8.1, 1.0 Hz, 1H), 7.82 (ddd, J = 9.8, 2.6, 1.5 Hz, 1H), 7.66-7.58 (m, 2H), 7.41 (ddd, J = 9.1, 1.7, 0.9 Hz, 1H), 7.18 (t, J = 7.5 Hz, 2H), 5.77 (s, 2H).

### Compound 20

¹H NMR (500 MHz, DMSO-d₆): δ = 10.61 (br s, 1H), 8.12 (t, J = 8.8 Hz, 2H), 7.51 (d, J = 9.0 Hz, 2H), 7.41 (t, J = 8.8 Hz, 2H), 6.90 (d, J = 9.0 Hz, 2H), 5.73 (s, 2H), 3.71 (s, 3H)

### Compound 21

¹H NMR (500 MHz, DMSO-d₆): δ = 10.92 (br s, 1H), 8.13 (t, J = 8.2 Hz, 2H), 7.70 (d, J = 9.1 Hz, 2H), 7.42 (t, J = 8.9 Hz, 2H), 7.35 (d, J = 8.6 Hz, 2H), 5.79 (s, 2H), 3.31 (s, 3H)

### Compound 22

¹H NMR (400 MHz, DMSO-d₆): δ = 10.49 (s, 1H), 7.66 (dt, J = 7.6, 1.0 Hz, 1H), 7.57 (dd, J = 2.6, 1.5 Hz, 1H), 7.49 (t, J = 8.0 Hz, 1H), 7.28 (d, J = 2.4 Hz, 1H), 7.12 (ddd, J = 8.3, 2.7, 0.9, 1H), 7.06 (dd, J = 8.7, 2.4, 1H), 6.91 (d, J = 8.7, 1H), 5.71 (s, 2H), 3.84 (s, 3H), 3.71 (s, 3H), 3.70 (s, 3H).

### Compound 23

¹H NMR (400 MHz, DMSO-d₆): δ = 10.65 (s, 1H), 7.66 (dt, J = 7.8, 1.1 Hz, 1H), 7.57 (dd, J = 2.5, 1.5 Hz, 1H), 7.52 (dd, J = 13.5, 2.5, 1H), 7.49 (t, J = 7.9 Hz, 1H), 7.27 (ddd, J = 9.9, 2.5, 1.4, 1H), 7.15 (t, J = 9.5, 1H), 7.12 (ddd, J = 8.3, 2.6, 0.9, 1H), 5.73 (s, 2H), 3.84 (s, 3H), 3.80 (s, 3H).

### Compound 24

¹H NMR (500 MHz, DMSO-d₆): δ = 10.54 (s, 1H), 7.66 (dt, J = 7.6, 1.3 Hz, 1H), 7.56 (dd, J = 2.5, 1.5 Hz, 1H), 7.48 (t, J = 8.1 Hz, 1H), 7.25 (d, J = 2.0, 1H), 7.12 (ddd, J = 8.3, 2.7, 0.9, 1H), 6.96 (dd, J = 8.5, 2.2, 1H), 6.88 (d, J = 8.4, 1H), 5.99 (s, 2H), 5.71 (s, 2H), 3.84 (s, 3H), 3.80 (s, 3H).

### Compound 25

¹H NMR (400 MHz, DMSO-d₆): δ = 10.65 (s, 1H), 7.65 (dd, J = 8.3, 2.0 Hz, 1H), 7.63-7.57 (m, 2H), 7.55 (d, J = 2.0 Hz, 1H), 7.18 (t, J = 8.9, 2H), 7.13 (d, J = 8.5 Hz, 1H), 5.71 (s, 2H), 3.84 (s, 3H), 3.82 (s, 3H).

### Compound 26

¹H NMR (400 MHz, DMSO-d₆): δ = 10.68 (s, 1H), 7.75 (dd, J = 7.9, 1.9 Hz, 1H), 7.67 (ddd, J = 8.4, 4.4, 2.0, 1H), 7.62-7.58 (m, 2H), 7.42 (dd, J = 11.4, 8.4, 1H), 7.18 (t, J = 8.9, 2H), 5.75 (s, 2H), 3.94 (s, 3H).

### Compound 27

¹H NMR (400 MHz, DMSO-d₆): δ = 10.69 (s, 1H), 7.77-7.70 (m, 2H), 7.62-7.56 (m, 2H), 7.50 (tt, J = 9.3, 2.3, 1H), 7.18 (t, J = 8.8, 2H), 5.78 (s, 2H).

### Compound 28

¹H NMR (500 MHz, DMSO-d₆): δ = 10.48 (br s, 1H), 8.01 (d, J = 8.6 Hz, 2H), 7.49 (d, J = 8.8 Hz, 2H), 7.11 (d, J = 8.6 Hz, 2H), 6.91 (d, J = 8.8 Hz, 2H), 5.67 (s, 2H), 3.82 (s, 3H), 3.71 (s, 3H); ¹³C NMR (125 MHz, DMSO-d₆) δ 164.1, 162.4, 161.0, 155.7, 131.3, 127.9, 120.9, 119.2, 114.7, 114.0, 55.3, 55.2.

### Compound 29

¹H NMR (500 MHz, DMSO-d₆): δ = 10.69 (br s, 1H), 8.23 (d, J = 8.1 Hz, 2H), 8.14 (d, J = 8.1 Hz, 2H), 7.61-7.50 (m, 4H), 7.18 (t, J = 8.8 Hz, 2H), 5.77 (s, 2H), 3.89 (s, 3H) ; ¹³C NMR (125 MHz, DMSO-d₆) δ 163.2, 162.8, 158.4 (d, J = 239 Hz),149.8, 134.5, 131.1, 128.5, 125.9, 121.8, 121.2, 121.2, 115.7, 115.5, 55.4.

### Compound 30

¹H NMR (500 MHz, DMSO-d₆): δ = 10.49 (br s, 1H), 8.20 (d, J = 8.4 Hz, 2H), 7.57 (d, J = 8.1 Hz, 2H), 7.49 (d, J = 8.8 Hz, 2H), 6.91 (d, J = 8.7 Hz, 2H), 5.73 (s, 2H), 3.72 (s, 3H)

### Compound 31

¹H NMR (500 MHz, DMSO-d₆): δ = 10.84 (br s, 1H), 8.20 (d, J = 8.5 Hz, 2H), 7.69 (d, J = 8.8 Hz, 2H), 7.57 (d, J = 8.2 Hz, 2H), 7.36 (d, J = 8.5 Hz, 3H), 5.80 (s, 2H) ; ¹³C NMR (125 MHz, DMSO-d₆) δ 163.2, 163.1, 149.8, 144.1, 137.3, 128.5, 125.9, 122.0, 121.9, 121.8, 121.1, 121.0, 120.8, 119.1, 119.0, 55.4.

### Compound 32

¹H NMR (500 MHz, DMSO-d₆): δ = 10.50 (br s, 1H), 8.23 (d, J = 8.2 Hz, 2H), 8.14 (d, J = 8.1 Hz, 2H), 7.49 (d, J = 8.8 Hz, 2H), 6.91 (d, J = 8.8 Hz, 2H), 5.75 (s, 2H), 3.72

### (s, 3H)

### Compound 33

¹H NMR (500 MHz, DMSO-d₆): δ = 10.70 (br s, 1H), 8.23 (d, J = 8.1 Hz, 2H), 8.14 (d, J = 8.1 Hz, 2H), 7.60 (dd, J = 8.1, 3.1 Hz, 2H), 7.18 (t, J = 8.6 Hz, 2H), 5.79 (s, 2 H), 3.89 (s, 3H)

### Compound 34

¹H NMR (500 MHz, DMSO-d₆): δ =10.86 (br s, 1H), 8.23 (d, J = 8.0 Hz, 2H), 8.14 (d, J = 8.1 Hz, 2H), 7.69 (d, J = 8.7 Hz, 2H), 7.36 (d, J = 8.5 Hz, 2H), 5.81 (s, 2H), 3.89 (s, 3H) ; ¹³C NMR (125 MHz, DMSO-d₆) δ 165.6, 163.4, 163.1, 144.1, 137.3, 131.3, 130.9, 130.2, 126.7, 121.8, 121.1, 120.8, 120.1, 55.5, 52.4.

### (Synthesis Example 35: Synthesis of Compound 35)

Compound 35 was synthesized in accordance with the following synthetic pathway.

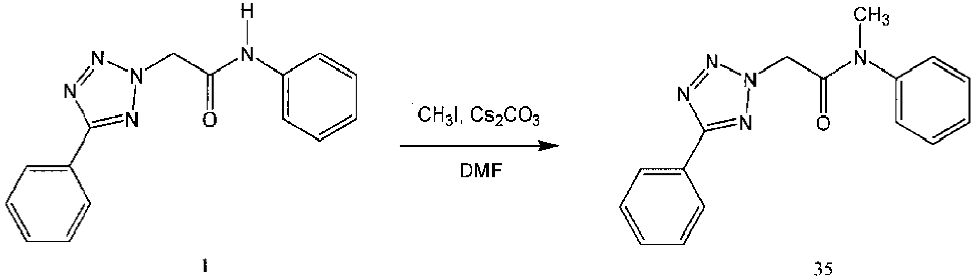

In a 50 mL eggplant-shaped flask, compound 1 (0.64 mmol) obtained in Synthesis Example 1 was dissolved in anhydrous DMF (15 mL) and cesium carbonate (343 mg, 0.97 mmol) was added thereto. The solution was stirred at room temperature for 10 minutes. Thereafter, methyl iodide (1.4 mmol) was added and the reaction solution was stirred at room temperature for 15 hours. The solvent was distilled away under reduced pressure and the obtained residue was separated into layers by use of water and ethyl acetate. The organic layer was washed with a saturated saline solution and dried over sodium sulfate. After the solvent was distilled away, the residue was purified by silica gel column chromatography. The obtained solid substance was recrystallized from a mixed solvent of n-hexane and chloroform to obtain compound 35 as a colorless prism crystal (yield: 73%).

The ¹H NMR spectrum of the resultant compound 35 is shown below.

### Compound 35

¹HNMR (500 MHz, CDCl₃): δ = 8.14 (dd, J = 9.5, 2.5 Hz, 2 H), 7.53(t, J = 9.0 Hz, 2 H),7.49-7.45 (m, 2 H), 7.36 (d, J = 9.0 Hz, 2 H), 5.23 (s, 2H), 4.98 (d, J = 6.2 Hz, 1H), 3.36 (s, 3H)

### (Synthesis Examples 36 to 57: Synthesis of compounds 36 to 57)

Compounds 36 to 57 were synthesized in the same operation as in Synthesis Example 35 except that compounds 2 to 10, 13 to 19, and 22 to 27 were used in place of compound 1.

The ¹H NMR spectra of compounds 36 to 57 obtained are shown below

### Compound 36

¹HNMR (500 MHz, CDCl₃): δ = 8.16-8.14 (m, 2H), 7.50-7.46 (m, 3H), 7.42-7.34 (m, 3H), 7.30 (d, J = 7.3 Hz, 1H), 5.21 (d, J = 6.3 Hz, 1H), 4.98 (d, J = 6.2 Hz, 1H), 3.30 (s, 3H), 2.41 (s, 3H) ; ¹³C NMR (125 MHz, CDCl₃) δ 165.3, 163.9, 140.1, 135.7, 132.1, 130.3, 129.6, 128.8, 128.2, 128.0, 127.3, 127.0, 53.9, 36.5, 17.4.

### Compound 37

¹HNMR (500 MHz, CDCl₃): δ = 8.16-8.14 (m, 2H), 7.50-7.46 (m, 3H), 7.40 (t, J = 8.1 Hz, 1H), 7.26 (d, J = 8.7 Hz, 1H), 7.16-7.155 (m, 2H), 5.25 (s, 2H), 3.35 (s, 3H), 2.43 (s, 3H) ; ¹³C NMR (125 MHz, CDCl₃) δ 165.3, 163.7, 141.6, 140.8, 130.3, 130.2, 129.8, 128.8, 127.8, 127.3, 127.0, 124.2, 54.2, 37.82, 21.3.

### Compound 38

¹HNMR (500 MHz, CDCl₃): δ = 8.16-8.13 (m, 2H), 7.50-7.46 (m, 3H), 7.32 (d, J = 8.1 Hz, 2H), 7.25 (d, J = 9.2 Hz, 2H), 5.23 (s, 2H), 3.34 (s, 3H), 2.42 (s, 3H) ; ¹³C NMR (125 MHz, CDCl₃) δ 165.3, 163.8, 139.2, 139.0, 131.0, 130.3, 128.8, 127.3, 127.0, 126.9, 54.11, 37.86, 21.11.

### Compound 39

¹HNMR (500 MHz, CDCl₃): δ = 8.16-8.14 (m, 2H), 7.50-7.45 (m, 3H), 7.43 (ddd, J = 9.2, 7.6, 1.6 Hz, 1H), 7.32 (dd, J = 8.2, 1.6 Hz, 1H), 7.09-7.05 (m, 2H), 5.24 (d, J = 16.1 Hz, 1H), 5.16 (d, J = 16.2 Hz, 1H), 3.95 (s, 3H), 3.27 (s, 3H) ; ¹³C NMR (125 MHz, CDCl₃) δ 165.3, 164.5, 155.0, 130.6, 130.2, 129.9, 129.0, 128.8, 127.4, 127.0, 121.7, 55.8, 53.9, 36.8.

### Compound 40

¹HNMR (500 MHz, CDCl₃): δ = 8.16-8.14 (m, 2H), 7.50-7.46 (m, 3H), 7.43 (t, J = 8.1 Hz, 1H), 6.99 (dd, J = 8.4, 2.0 Hz, 1H), 6.95 (ddd, J = 7.8, 1.9, 0.8 Hz, 1H), 6.88 (t, J = 2.2 Hz, 1H), 5.28 (s, 2H), 3.87 (s, 3H), 3.35 (s, 3H) ; ¹³C NMR (125 MHz, CDCl₃) δ 165.3, 163.7, 161.1, 142.8, 131.2, 130.3, 128.8, 127.3, 127.0, 119.3, 114.5, 113.1, 55.6, 54.1, 37.8.

### Compound 41

¹HNMR (500 MHz, CDCl₃): δ = 8.16-8.13 (m, 2H), 7.50-7.46 (m, 3H), 7.28 (d, J = 8.8 Hz, 2H), 7.01 (d, J = 8.9 Hz, 2H), 5.23 (s, 2H), 3.85 (s, 3H), 3.33 (s, 3H) ; ¹³C NMR (125 MHz, CDCl₃) δ 165.3, 164.0, 159.8, 134.2, 130.3, 128.8, 128.4, 127.3, 127.0, 115.5, 55.6, 54.1, 38.0.

### Compound 42

¹HNMR (500 MHz, CDCl₃): δ = 8.15-8.13 (m, 2H), 7.85 (dd, J = 7.7, 1.1 Hz, 1H), 7.76 (dt, J = 7.8, 1.3 Hz, 1H), 7.59 (t, J = 7.7 Hz, 1H), 7.52-7.47 (m, 4H), 5.25 (d, J = 2.0 Hz, 2H), 3.43 (s, 3H) ; ¹³C NMR (125 MHz, CDCl₃) δ 165.5, 163.2, 144.1, 135.0, 134.6, 130.4, 129.8, 129.3, 128.8, 127.0, 126.9, 115.2, 112.7, 53.8, 37.7.

### Compound 43

¹HNMR (500 MHz, CDCl₃): δ = 7.92 (s, 3H), 7.52-7.42 (m, 5 H), 7.05 (s, 1H), 5.03 (s, 2H), 3.16 (s, 3H); ¹³C NMR (125 MHz, CDCl₃) δ 165.5, 163.4, 142.6, 132.6, 132.0131.6, 130.9, 130.5, 128.9, 127.0, 126.9, 117.1, 114,9, 54.0, 38.1.

### Compound 44

¹HNMR (500 MHz, CDCl₃): δ = 8.13 (dd, J = 7.7, 2.0 Hz, 2H), 7.82 (d, J = 8.1 Hz, 2 H), 7.52-7.49 (m, 5H), 5.29 (s, 2H), 3.40 (s, 3H); ¹³C NMR (125 MHz, CDCl₃) δ 165.5, 163.3, 145.7, 134.3, 130.5, 128.9, 128.1, 127.0, 126.9, 117,5, 54.0, 37.9.

### Compound 45

¹HNMR (500 MHz, CDCl₃): δ = 8.08 (dd, J = 6.9, 2.1 Hz, 1H), 7.53 (t, J = 7.9 Hz, 2H), 7.45 (dt, J = 7.9, 1.6 Hz, 2H), 7.38 (dd, J = 7.2, 1.4 Hz, 2H), 7.09-7.04 (m, 2H), 5.27 (s, 2H), 3.93 (s, 3H), 3.36 (s, 3H) ; ¹³C NMR (125 MHz, CDCl₃) δ 163.7, 163.3, 157.4, 141.7, 131.5, 130.9, 130.4, 129.0, 127.3, 120.7, 116.4, 111.8, 56.0, 54.1, 37.8.

### Compound 46

¹HNMR (500 MHz, CDCl₃): δ = 7.74 (d, J = 7.6 Hz, 1H), 7.69 (t, J = 1.5 Hz, 1H), 7.54 (t, J = 7.2 Hz, 2H), 7.48 (t, J = 7.3 Hz, 1H), 7.40-7.36 (m, 3H), 7.02 (dd, J = 8.1, 2.8, 1.1 Hz, 1H), 5.24 (s, 2H), 3.89 (s, 3H), 3.37 (s, 3H) ; ¹³C NMR (125 MHz, DCl₃) δ 165.3, 163.7, 159.9, 141.7, 130.5, 129.9, 129.1, 128.5, 127.3, 119.4, 116.9, 111.5, 55.4, 54.1, 37.9.

### Compound 47

¹HNMR (500 MHz, CDCl₃): δ = 8.08 (d, J = 9.0 Hz, 2H), 7.53 (t, J = 7.9 Hz, 2H), 7.46 (tt, J = 7.4, 1.5 Hz, 1H), 7.36 (dd, J = 7.2, 1.3 Hz, 2H), 6.99 (d, J = 8.1 Hz, 2H), 5.22 (s, 2H), 3.87 (s, 3H), 3.37 (s, 3H) ; ¹³C NMR (125 MHz, CDCl₃) δ 165.2, 163.8, 161.2, 141.7, 130.5, 129.1, 128.5, 127.3, 119.9, 114.2, 55.3, 54.0, 37.9.

### Compound 48

¹HNMR (500 MHz, CDCl₃): δ = 7.74 (dt, J = 7.7, 1.2 Hz, 1H), 7.69 (dd, J = 2.5, 1.5 Hz, 1H), 7.39 (t, J = 7.9 Hz, 1H), 7.28 (d, J = 8.9 Hz, 2H), 7.03-7.00 (m, 3H), 5.23 (s, 2H), 3.89 (s, 3H), 3.86 (s, 3H), 3.33 (s, 3 H) ; ¹³C NMR (125 MHz, CDCl₃) δ 165.2, 164.0, 159.9, 159.8, 134.2, 129.9, 128.5, 128.4, 119.4, 116.9, 115.6, 111.5, 55.6, 55.4, 54.2, 38.0.

### Compound 49

¹HNMR (500 MHz, CDCl₃): δ = 7.74 (dt, J = 7.7, 1.2 Hz, 2H), 7.69 (dd, J = 2.5, 1.5 Hz, 2H), 7.41-7.35 (m, 3H), 7.22 (t, J = 8.7 Hz, 1H), 7.02 (ddd, J = 8.3, 2.6, 1.0 Hz, 2H), 5.22 (s, 2H), 3.89 (s, 3H), 3.45 (s, 3H) ; ¹³C NMR (125 MHz, CDCl₃) δ 165.3, 163.7,162.4 (d, J = 249 Hz), 159.9, 137.6, 129.9, 129.3, 129.2, 128.4, 119.4, 117.7, 117.5, 116.9, 111.5, 55.4, 54.1, 38.1.

### Compound 50

¹HNMR (500 MHz, CDCl₃): δ = 7.74 (dt, J = 7.9, 1.2 Hz, 1H), 7.85 (ddd, J = 9.6, 2.6, 1.1 Hz, 2H), 7.46 (td, J = 8.1, 5.8 Hz, 1H), 7.28 (d, J = 9.7 Hz, 1H), 7.16 (ddd, J = 7.5, 2.7, 0.9 Hz, 1H), 7.02 (d, J = 8.9 Hz, 2H), 5.23 (s, 2H), 3.86 (s, 3H), 3.33 (s, 3H) ; ¹³C NMR (125 MHz, CDCl₃) δ 164.4, 163.9, 163.1(d, J = 298 Hz), 159.8, 134.2, 130.5(d, J = 8.8 Hz), 129.3(d, J = 7.5 Hz), 128.44, 122.6(d, J = 2.5 Hz), 117.2(d, J = 21.3 Hz), 115.6, 114.0(d, J = 23.8 Hz), 55.6, 54.2, 38.0.

### Compound 51

¹HNMR (500 MHz, CDCl₃): δ = 7.95 (dt, J = 7.8, 1.1 Hz, 1H), 7.85 (ddd, J = 9.5, 2.4, 0.9 Hz, 1H), 7.46 (td, J = 8.1, 5.8 Hz, 1H), 7.38-7.35(m, 2H), 7.23 (t, J = 8.5 Hz, 1H), 7.17 (ddd, J = 8.5, 2.6, 0.9 Hz, 1H), 5.23 (s, 2 H), 3.35 (s, 3H) ; ¹³C NMR (125 MHz, CDCl₃) δ 164.4, 164.0(d, J = 123 Hz), 163.8(d, J = 38 Hz), 161.8(d, J = 61 Hz), 137.6(d, J = 4 Hz), 130.5(d, J = 8 Hz), 129.3(d, J = 9 Hz),128.44, 122.6(d, J = 4 Hz), 117.6(d, J = 23 Hz), 117.3(d, J = 20 Hz), 115.6, 114.0(d, J = 25 Hz), 54.1, 38.1.

### Compound 52

¹H NMR (500 MHz, CDCl₃): δ = 7.73 (d, J = 7.6, Hz, 1H), 7.68 (s, 1H), 7.34 (t, J = 8.0 Hz, 1H), 7.01 (dd, J = 8.1, 1.9 Hz, 1H), 6.95-6.91 (m, 2H), 6.84 (d, J = 1.4, 1H), 5.27 (s, 2H), 3.93 (s, 3H), 3.93 (s, 3 ), 3.89 (s, 3H), 3.34 (s, 3H).

### Compound 53

¹H NMR (500 MHz, CDCl₃): δ = 7.66 (dt, J = 7.6, 1.0 Hz, 1H), 7.68 (dd, J = 2.5, 1.5 Hz, H), 7.39 (t, J = 8.0, 1H), 7.13 (dd, J = 11.4, 2.5 Hz, 1H), 7.11 (dd, J = 8.5, 2.4, 1H), 7.07 (t, J = 8.6, 1H), 7.03 (ddd, J = 7.5, 2.7, 0.9, 1H), 5.25 (s, 2H), 3.94 (s, 3H), 3.89 (s, 3H), 3.32 (s, 3H).

### Compound 54

¹H NMR (500 MHz, CDCl₃): δ = 7.62 (dt, J = 8.0, 2.0 Hz, 1H), 7.53 (dd, J = 2.5, 2.0 Hz, 1H), 7.48 (t, J = 8.0 Hz, 1H), 7.21 (d, J = 2.0, 1H), 7.11 (ddd, J = 8.5, 2.5, 0.9, 1H), 7.05 (dd, J = 8.0, 2.0, 1H), 7.01 (d, J = 8.0, 1H), 6.09 (s, 2H), 5.46 (s, 2H), 3.83 (s, 3H), 3.16 (s, 3H).

### Compound 55

¹H NMR (500 MHz, CDCl₃): δ = 7.74 (dt, J = 8.4, 1.2 Hz, 1H), 7.65 (s, 1H), 7.38-7.33 (m, 2H), 7.24-7.19 (m, 2H), 6.96 (d, J = 8.3 Hz, 1H), 5.21 (s, 2H), 3.97 (s, 3H), 3.95 (s, 3H), 3.34 (s, 3H).

### Compound 56

¹H NMR (500 MHz, CDCl₃): : δ = 7.77 (dd, J = 8.1, 1.9 Hz, 1H), 7.70 (ddd, J = 8.4, 4.4, 2.0, 1H), 7.39-7.34 (m, 2H), 7.23 (t, J = 6.9, 2H), 7.18 (dd, J = 10.9, 8.4, 1H), 5.22 (s, 2H), 3.98 (s, 3H), 3.35 (s, 3H).

### Compound 57

¹H NMR (500 MHz, CDCl₃): δ = 7.72-7.66 (m, 2H), 7.40-7.35 (m, 2H), 7.24 (t, J = 8.7, 2H), 6.92 (tt, J = 8.8, 2.4, 1H), 5.23 (s, 2H), 3.35 (s, 3H).

### (Synthesis Example 58: Synthesis of Compound 58)

Compound 58 was synthesized in the same operation as in Synthesis Example 35 except that ethyl iodide was used in place of the methyl iodide.

The ¹H NMR spectrum of compound 58 obtained is shown below.

### Compound 58

¹HNMR (500 MHz, CDCl₃): δ = 8.15-8.13 (m, 2H), 7.53 (dd, J = 9.0, 2.0 Hz, 2H), 7.50-7.45 (m, 4H), 7.33 (dd, J = 9.0, 1.5 Hz, 2H), 5.17 (s, 2H), 3.83 (q, J = 7.2 Hz, 2H), 1.17 (t, J = 7.2 Hz 3H).

### (Synthesis Examples 59 to 65: Synthesis of Compounds 59 to 65)

Compounds 59 to 65 were synthesized in the same operation as in Synthesis Example 35 except that compounds 7, 11, 12, 28, 29, 31 and 34 were used in place of compound 1, and ethyl iodide was used in place of methyl iodide.

The ¹H NMR spectra of compounds 59 to 65 obtained are shown below.

### Compound 59

¹HNMR (400 MHz, CDCl₃): δ = 8.15-8.13 (m, 2H), 7.50-7.46 (m, 3H), 7.24 (d, J = 8.9 Hz, 2H), 7.01 (d, J = 9.1 Hz, 2H), 5.18 (s, 2H), 3.85(s, 3H), 3.79 (q, J = 7.2 Hz, 2H), 1.16 (t, J = 7.2 Hz, 3H).

### Compound 60

¹HNMR (400 MHz, CDCl₃): δ = 8.16-8.13 (m, 2H), 7.50-7.47 (m, 3H), 7.35-7.32 (m, 2H), 7.23 (t, J = 8.8 Hz, 2H), 5.17 (s, 2H), 3.81 (q, J = 7.2 Hz, 2H), 1.17 (t, J = 7.2 Hz, 3H).

### Compound 61

¹HNMR (400 MHz, CDCl₃): δ = 8.15-8.13 (m, 2H), 7.50-7.46 (m, 3H), 7.39 (s, 4H), 5.19 (s, 2H), 3.82 (q, J = 7.2 Hz, 2H), 1.18 (t, J = 7.2 Hz, 3H).

### Compound 62

¹HNMR (400 MHz, CDCl₃): δ = 8.07 (d, J = 8.8 Hz, 2H), 7.24 (d, J = 8.8 Hz, 2H), 7.00 (t, J = 8.0, 4H), 5.16 (s, 2H), 3.8 7(s, 3H), 3.85 (s, 3H), 3.78 (q, J = 7.2 Hz, 2H), 1.17 (t, J = 7.2 Hz, 3H).

### Compound 63

¹HNMR (400 MHz, CDCl₃): δ = 8.18 (d, J = 8.9 Hz, 2H), 7.35-7.32 (m, 4H), 7.24 (t, J = 8.0, 2H), 5.17 (s, 2H), 3.80 (q, J = 7.2 Hz, 2H), 1.17 (t, J = 7.2 Hz, 3H).

### Compound 64

¹HNMR (400 MHz, CDCl₃): δ = 8.19 (d, J = 8.8 Hz, 2H), 7.40 (s, 4H), 7.33 (d, J = 8.1 Hz, 4H), 5.17 (s, 2H), 3.81 (q, J = 7.2 Hz, 2H), 1.17 (t, J = 7.2 Hz, 3H).

### Compound 65

¹HNMR (400 MHz, CDCl₃): : δ = 8.22 (d, J = 8.5 Hz, 2H), 8.16 (d, J = 8.3 Hz, 2H), 7.40 (s, 4H), 5.20 (s, 2H), 3.96 (s, 3H), 3.82 (q, J = 7.1 Hz, 2H), 1.1 8(t, J = 7.2 Hz, 3H) .

### (Synthesis Example 66: Synthesis of Compound 66)

Compound 66 was synthesized in the same operation as in Synthesis Example 35 except that n-propyl iodide was used in place of methyl iodide.

The ¹H NMR spectrum of compound 66 obtained is shown below.

### Compound 66

¹HNMR (500 MHz, CDCl₃): δ = 8.03 (dd, J = 7.8, 1.4 Hz, 2H), 7.58-7.52 (m, 7H), 7.48-7.44 (m, 1H), 5.34 (s, 2H), 3.65 (t, J = 7.4 Hz, 2H), 1.43 (sext, J = 7.4 Hz, 2H), 0.83 (t, J = 7.4 Hz 3H).

### (Synthesis Example 67: Synthesis of compound 67)

In a 50 mL eggplant-shaped flask, compound 48 (1.0 mmol) was dissolved in THF (10 mL) and Lawesson reagent (0.55 mmol) was added thereto. After the reaction solution was stirred at 80°C for 4 hours, the solvent was distilled away under reduced pressure. The residue obtained was purified by silica gel column chromatography. The solid substance obtained was recrystallized from a mixed solvent of n-hexane and chloroform to obtain compound 67 as colorless prism crystal (yield: 99%).

### (Synthesis Examples 68 to 70: Synthesis of compounds 68 to 70)

Compounds 68 to 70 were synthesized in the same operation as in Synthesis Example 67 except that compounds 49 to 51 were used in place of compound 48.

The ¹H NMR spectra of compounds 67 to 70 obtained in Synthesis Examples 67 to 70 are shown below.

### Compound 67

¹HNMR (500 MHz, CDCl₃): δ = 7.74 (dt, J = 7.7, 1.2 Hz, 1H), 7.69 (dd, J = 2.4, 1.5 Hz, 1H), 7.39 (t, J = 7.9 Hz, 1H), 7.24 (d, J = 9.0 Hz, 2H), 7.02 (ddd, J = 6.7, 2.6, 0.9 Hz, 1H), 7.00 (d, J = 8.9 Hz, 2H), 5.46 (s, 2H), 3.89 (s, 3H), 3.84 (s, 3 H), 3.76(s, 3H) ; ¹³C NMR (125 MHz, CDCl₃) δ 193.2, 164.0, 160.0, 159.9, 136.8, 129.2, 128.5, 126.6, 119.4, 116.9, 115.6, 111.5, 60.8, 55.6, 55.4, 46.3.

### Compound 68

¹HNMR (500 MHz, CDCl₃): δ = 7.74 (dt, J = 7.6, 1.1 Hz, 1H), 7.69 (dd, J = 2.5, 1.0 Hz, 1H), 7.39 (t, J = 7.9 Hz, 1H), 7.34-7.31 (m, 2H), 7.22 (t, J = 7.1 Hz, 2H), 7.03 (ddd, J = 8.3, 2.7, 0.9 Hz, 1H), 5.46 (s, 2H), 3.89 (s, 3H), 3.84 (s, 3H), 3.76 (s, 3H) ; ¹³C NMR (125 MHz, CDCl₃) δ 193.1, 165.1, 162.5(d, J = 251 Hz), 159.9, 140.1, 130.0, 128.4, 127.6, 127.5, 119.4, 117.7, 117.6, 116.9, 111.6, 60.8, 55.4, 46.3.

### Compound 69

¹HNMR (500 MHz, CDCl₃): δ = 7.95 (dt, J = 8.0, 1.2 Hz, 1H), 7.85 (ddd, J = 9.6, 2.6, 1.1 Hz, 1H), 7.46 (dt, J = 8.0, 5.7 Hz, 1H), 7.25 (d, J = 8.9 Hz, 2H), 7.17 (ddd, J = 8.4, 2.6, 0.9 Hz, 1H), 7.02 (d, J = 8.9 Hz, 2H), 5.46 (s, 2H), 3.85 (s, 3H), 3.76 (s, 3H) ; ¹³C NMR (125 MHz, CDCl₃) δ 193.1, 164.2, 163.9, 160.0, 136.7, 130.5, 129.3, 126.6, 122.7, 117.2, 115.6, 114.0, 60.8, 55.6, 46.3.

### Compound 70

¹HNMR (500 MHz, CDCl₃): δ = 7.95 (dt, J = 7.9, 1.2 Hz, 1H), 7.85 (ddd, J = 9.5, 2.6, 1.1 Hz, 1H), 7.47 (td, J = 8.1, 5.8 Hz, 1H), 7.36-7.32 (m, 2H), 7.23 (t, J = 8.0 Hz, 1H), 7.17 (ddd, J = 8.4, 2.6, 1.0 Hz, 1H), 5.46 (s, 2H), 3.77 (s, 3H) ; ¹³C NMR (125 MHz, CDCl₃) δ 193.0, 164.2, 163.7, 161.7, 140.0, 130.6, 129.2, 127.5, 122.7, 117.7, 117.3, 114.0, 60.7, 46.3.

### (Synthesis Examples 71 and 72: Synthesis of compounds 71 and 72)

Compounds 71 and 72 were synthesized (R² = -H or 4-OMe) in accordance with the following synthetic pathway.

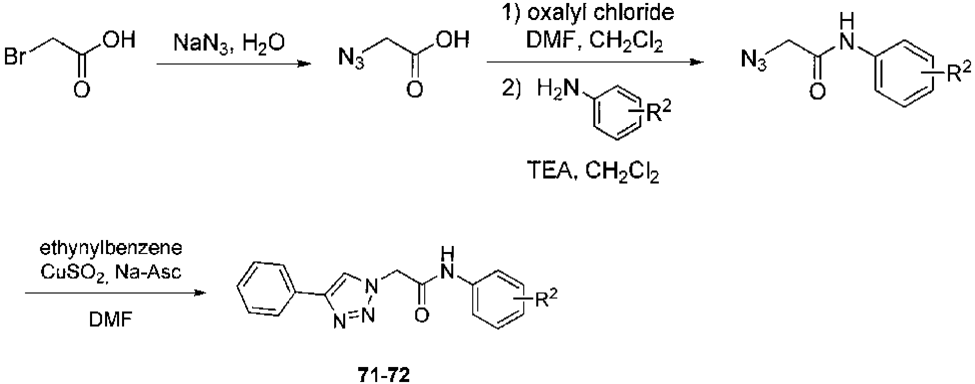

### (1) Synthesis of Azideacetic Acid

In a 50 mL eggplant-shaped flask, sodium azide (1.58 g, 2.43 mmol) was dissolved in water (15 mL). The solution was stirred at 0°C. To the solution, bromoacetic acid (2.2 g, 16.2 mmol) dissolved in water (10 mL) was gently added dropwise. The reaction solution was stirred at room temperature for 24 hours. The reaction solution was cooled again to 0°C and 2 N hydrochloric acid was added little by little until pH = 2 was obtained. The reaction solution was extracted with diethyl ether and the extract was dried over sodium sulfate. The solvent was distilled away under reduced pressure to obtain azideacetic acid (1.96 g) as a colorless liquid (yield: 99%).

### (2) Synthesis of Azide Derivative

In a 20 mL eggplant-shaped flask, the azideacetic acid (200 mg, 1.98 mmol) obtained in the above (1) was dissolved in dichloromethane (5 mL). To the solution, a single drop of DMF was added. The reaction solution was stirred at 0°C. To the reaction solution, oxalyl chloride (326 mg, 2.57 mmol) dissolved in dichloromethane was added dropwise. The reaction solution was stirred at 0°C to room temperature for 3 hours. The solvent was distilled away under reduced pressure and the obtained liquid was dissolved in dichloromethane (4 mL). The reaction solution was stirred at 0°C. To the resultant reaction solution, aniline (Synthesis Example 71) dissolved in dichloromethane or 4-methoxyaniline (Synthesis Example 72) (2.18 mmol) and triethylamine (400 mg, 3.95 mmol) were added dropwise. The resultant reaction solution was stirred at 0°C to room temperature. Fifteen hours later, water was added to the reaction solution. The resultant reaction solution was stirred at room temperature for one hour and separated into layers by use of water and ethyl acetate. The organic layer was washed with 2 N hydrochloric acid and a saturated saline solution, and dried over sodium sulfate. The solvent was distilled away under reduced pressure to obtain an azide derivative as a light-yellow solid substance (yield: 72 to 73%).

### (3) Synthesis of Compounds 71 and 72

In a 30 mL eggplant-shaped flask, the azide derivative (0.73 mmol) obtained in the above (2) and ethynylbenzene (82 mg, 0.80 mol) were dissolved in DMF (4 mL), further copper sulfate pentahydrate (9 mg, 0.037 mmol) and sodium ascorbate (14.4 mg, 0.073 mmol) were added thereto, and then, water (1 mL) was added. The reaction solution was stirred at 60°C for 20 hours and separated into layers by use of water and ethyl acetate. The organic layer was washed with an aqueous sodium thiosulfate solution and dried over sodium sulfate. Thereafter the solvent was distilled away under reduced pressure, and the obtained residue was purified by silica gel column chromatography. The solid substance obtained was recrystallized from a mixed solvent of n-hexane and ethyl acetate to obtain compounds 71 and 72 as colorless needle-like crystals (yield: 44 to 73%).

The ¹H NMR spectra of compounds 71 and 72 obtained are shown below.

### Compound 71

¹H NMR (500 MHz, acetone-d₆): δ = 9.64 (br s, 1H), 8.43 (s, 1H), 7.93 (dd, J = 8.5, 1.0 Hz, 2H), 7.67 (d, J = 8.5 Hz, 2H), 7.44 (t, J = 7.0 Hz, 2H), 7.33 (t, J = 7.5 Hz, 3H), 7.11 (tt, J = 7.5, 1.0 Hz, 1H), 5.42 (s, 2H); ¹³C NMR (126 MHz, CDCl₃) δ 164.9, 148.0, 139.5, 132.4, 129.8, 129.7, 128.7, 126.3, 125.0, 123.2, 120.3, 53.6.

### Compound 72

¹H NMR (500 MHz, DMSO-d₆): δ = 10.38 (s, 1H), 8.59 (s, 1H), 7.93 (dd, J = 8.3, 1.3 Hz, 2H), 7.50 (d, J = 9.1 Hz, 2H), 7.46 (t, J = 7.5 Hz, 2H), 7.34 (tt, J = 7.5, 1.0 Hz, 1H), 6.91 (d, J = 9.1 Hz, 2H), 5.43 (s, 2H), 3.72 (s, 3H); ¹³C NMR (126 MHz, CDCl₃) δ 163.6, 155.6, 146.2, 131.5, 128.9, 127.9, 125.1, 123.0, 120.8, 114.0, 55.2, 52.3.

### (Synthesis Examples 73 and 74: Synthesis of compounds 73 and 74)

In a 20 mL eggplant-shaped flask, compound 71 (Synthesis Example 73) or compound 72 (Synthesis Example 74) (0.14 mmol) was dissolved in anhydrous DMF (2.5 mL). To the solution, cesium carbonate (75 mg, 0.23 mmol) was added. The reaction solution was stirred at room temperature for 10 minutes. Thereafter, methyl iodide (excessive amount) was added and the reaction solution was stirred at 60°C for 4 hours. The solvent was distilled away under reduced pressure and the residue obtained was separated into layers by use of water and dichloromethane. The organic layer was dried over sodium sulfate. After the solvent was distilled away, the obtained residue was purified by silica gel column chromatography. The resultant solid substance was recrystallized from a mixed solvent of n-hexane, and chloroform to obtain compound 73 and 74 as a light-yellow needle-like crystal (yield: 73%).

### (Synthesis Example 75: Synthesis of compound 75)

Compound 75 was synthesized in the same operation as in Synthesis Example 74 except that ethyl iodide was used in place of the methyl iodide.

The ¹H NMR spectra of compounds 73 to 75 obtained in Synthesis Examples 73 to 75 are shown below.

### Compound 73

¹H NMR (500 MHz, CDCl₃): δ = 7.99 (s, 1H), 7.93 (dd, J = 8.5, 1.0 Hz, 2H), 7.52 (t, J = 8.5 Hz, 2H), 7.44 (t, J = 7.2 Hz, 2H), 7.46 (tt, J = 7.5, 1.0 Hz, 1H), 7.42 (t, J = 7.5 Hz, 2H), 7.34-7.32 (m, 3H), 4.97 (s, 2H), 3.35 (s, 3H); ¹³C NMR (126 MHz, CDCl₃) δ 165.1, 148.1, 142.1, 130.9, 130.7, 129.3, 129.0, 128.3, 127.5, 126.0, 121.6, 51.5, 38.0.

### Compound 74

¹H NMR (500 MHz, CDCl₃): δ = 7.98 (s, 1H), 7.83 (dd, J = 8.6, 1.2 Hz, 2H), 7.41 (t, J = 7.4 Hz, 2H), 7.32 (tt, J = 7.4, 1.8 Hz, 1H), 7.22 (d, J = 8.9 Hz, 2H), 7.00 (d, J = 8.9 Hz, 2H), 4.96 (s, 2H), 3.85 (s, 3H), 3.31 (s, 3H); ¹³C NMR (126 MHz, CDCl₃) δ 165.4, 160.0, 148.0, 134.6, 130.9, 129.0, 128.6, 128.3, 121.6, 115.8, 55.8, 51.4, 38.2.

### Compound 75

¹H NMR (500 MHz, CDCl₃): δ = 7.98 (s, 1H), 7.83 (dd, J = 8.6, 1.2 Hz, 2H), 7.41 (t, J = 7.4 Hz, 2H), 7.32 (tt, J = 7.4, 1.8 Hz, 1H), 7.19 (d, J = 8.9 Hz, 2H), 7.00 (d, J = 8.9 Hz, 2H), 4.91 (s, 2 H), 3.86 (s, 3H), 3.77 (q, J = 7.2 Hz, 2H), 1.15 (t, J = 7.2 Hz, 2H); ¹³C NMR (126 MHz, CDCl₃) δ 164.9, 160.1, 148.0, 132.8, 130.9, 129.7, 129.0, 128.2, 126.0, 121.7, 115.7, 55.8, 51.7, 45.0, 13.0.

With respect to compounds 1 to 75 obtained in Synthesis Examples 1 to 75, reference symbols in the formula (α1) are as shown in Tables 8 to 12.

**[Table 8]**

| Number | Q1 | Q2 | R1 | R2 | R3 | X | Y |
|---|---|---|---|---|---|---|---|
| 1 | Benzene ring | Benzene ring | H | H | H | O | N |
| 2 | Benzene ring | Benzene ring | H | 2-Me | H | O | N |
| 3 | Benzene ring | Benzene ring | H | 3-Me | H | O | N |
| 4 | Benzene ring | Benzene ring | H | 4-Me | H | O | N |
| 5 | Benzene ring | Benzene ring | H | 2-OMe | H | O | N |
| 6 | Benzene ring | Benzene ring | H | 3-OMe | H | O | N |
| 7 | Benzene ring | Benzene ring | H | 4-OMe | H | O | N |
| 8 | Benzene ring | Benzene ring | H | 2-CN | H | O | N |
| 9 | Benzene ring | Benzene ring | H | 3-CN | H | O | N |
| 10 | Benzene ring | Benzene ring | H | 4-CN | H | O | N |
| 11 | Benzene ring | Benzene ring | H | 4-F | H | O | N |
| 12 | Benzene ring | Benzene ring | H | 4-OCF3 | H | O | N |
| 13 | Benzene ring | Benzene ring | 2-OMe | H | H | O | N |
| 14 | Benzene ring | Benzene ring | 3-OMe | H | H | O | N |
| 15 | Benzene ring | Benzene ring | 4-OMe | H | H | O | N |

**[Table 9]**

| Number | Q1 | Q2 | R1 | R2 | R3 | X | Y |
|---|---|---|---|---|---|---|---|
| 16 | Benzene ring | Benzene ring | 3-OMe | 4-OMe | H | O | N |
| 17 | Benzene ring | Benzene ring | 3-OMe | 4-F | H | O | N |
| 18 | Benzene ring | Benzene ring | 3-F | 4-OMe | H | O | N |
| 19 | Benzene ring | Benzene ring | 3-F | 4-F | H | O | N |
| 20 | Benzene ring | Benzene ring | 4-F | 4-OMe | H | O | N |
| 21 | Benzene ring | Benzene ring | 4-F | 4-OCF3 | H | O | N |
| 22 | Benzene ring | Benzene ring | 3-OMe | 3-OMe, 4-OMe | H | O | N |
| 23 | Benzene ring | Benzene ring | 3-OMe | 3-F, 4-OMe | H | O | N |
| 24 | Benzene ring | Benzodioxol ring | 3-OMe | H | H | O | N |
| 25 | Benzene ring | Benzene ring | 3-OMe, 4-OMe | 4-F | H | O | N |
| 26 | Benzene ring | Benzene ring | 3-OMe, 4-F | 4-F | H | O | N |
| 27 | Benzene ring | Benzene ring | 3-F, 5-F | 4-F | H | O | N |
| 28 | Benzene ring | Benzene ring | 4-OMe | 4-OMe | H | O | N |
| 29 | Benzene ring | Benzene ring | 4-OCF3 | 4-F | H | O | N |
| 30 | Benzene ring | Benzene ring | 4-OCF3 | 4-OMe | H | O | N |
| 31 | Benzene ring | Benzene ring | 4-OCF3 | 4-OCF3 | H | O | N |
| 32 | Benzene ring | Benzene ring | 4-CO2Me | 4-OMe | H | O | N |
| 33 | Benzene ring | Benzene ring | 4-CO2Me | 4-F | H | O | N |
| 34 | Benzene ring | Benzene ring | 4-CO2Me | 4-OCF3 | H | O | N |

**[Table 10]**

| Number | Q1 | Q2 | R1 | R2 | R3 | X | Y |
|---|---|---|---|---|---|---|---|
| 35 | Benzene ring | Benzene ring | H | H | Me | O | N |
| 36 | Benzene ring | Benzene ring | H | 2-Me | Me | O | N |
| 37 | Benzene ring | Benzene ring | H | 3-Me | Me | O | N |
| 38 | Benzene ring | Benzene ring | H | 4-Me | Me | O | N |
| 39 | Benzene ring | Benzene ring | H | 2-OMe | Me | O | N |
| 40 | Benzene ring | Benzene ring | H | 3-OMe | Me | O | N |
| 41 | Benzene ring | Benzene ring | H | 4-OMe | Me | O | N |
| 42 | Benzene ring | Benzene ring | H | 2-CN | Me | O | N |
| 43 | Benzene ring | Benzene ring | H | 3-CN | Me | O | N |
| 44 | Benzene ring | Benzene ring | H | 4-CN | Me | O | N |
| 45 | Benzene ring | Benzene ring | 2-OMe | H | Me | O | N |
| 46 | Benzene ring | Benzene ring | 3-OMe | H | Me | O | N |
| 47 | Benzene ring | Benzene ring | 4-OMe | H | Me | O | N |
| 48 | Benzene ring | Benzene ring | 3-OMe | 4-OMe | Me | O | N |
| 49 | Benzene ring | Benzene ring | 3-OMe | 4-F | Me | O | N |
| 50 | Benzene ring | Benzene ring | 3-F | 4-OMe | Me | O | N |
| 51 | Benzene ring | Benzene ring | 3-F | 4-F | Me | O | N |
| 52 | Benzene ring | Benzene ring | 3-OMe | 3-OMe, 4-OMe | Me | O | N |
| 53 | Benzene ring | Benzene ring | 3-OMe | 3-OMe. 4-F | Me | O | N |
| 54 | Benzene ring | Benzodioxol ring | 3-OMe | H | Me | O | N |
| 55 | Benzene ring | Benzene ring | 3-OMe, 4-OMe | 4-F | Me | O | N |
| 56 | Benzene ring | Benzene ring | 3-OMe. 4-F | 4-F | Me | O | N |
| 57 | Benzene ring | Benzene ring | 3-F. 5-F | 4-F | Me | O | N |

**[Table 11]**

| Number | Q1 | Q2 | R1 | R2 | R3 | X | Y |
|---|---|---|---|---|---|---|---|
| 58 | Benzene ring | Benzene ring | H | H | Et | O | N |
| 59 | Benzene ring | Benzene ring | H | 4-OMe | Et | O | N |
| 60 | Benzene ring | Benzene ring | H | 4-F | Et | O | N |
| 61 | Benzene ring | Benzene ring | H | 4-OCF3 | Et | O | N |
| 62 | Benzene ring | Benzene ring | 4-OMe | 4-OMe | Et | O | N |
| 63 | Benzene ring | Benzene ring | 4-OCF3 | 4-F | Et | O | N |
| 64 | Benzene ring | Benzene ring | 4-OCF3 | 4-OCF3 | Et | O | N |
| 65 | Benzene ring | Benzene ring | 4-CO2Me | 4-OCF3 | Et | O | N |
| 66 | Benzene ring | Benzene ring | H | H | n-Pr | O | N |

**[Table 12]**

| Number | Q1 | Q2 | R1 | R2 | R3 | X | Y |
|---|---|---|---|---|---|---|---|
| 67 | Benzene ring | Benzene ring | 3-OMe | 4-OMe | Me | S | N |
| 68 | Benzene ring | Benzene ring | 3-OMe | 4-F | Me | S | N |
| 69 | Benzene ring | Benzene ring | 3-F | 4-OMe | Me | S | N |
| 70 | Benzene ring | Benzene ring | 3-F | 4-F | Me | S | N |
| 71 | Benzene ring | Benzene ring | H | H | H | O | CH |
| 72 | Benzene ring | Benzene ring | H | 4-OMe | H | S | CH |
| 73 | Benzene ring | Benzene ring | H | H | Me | O | CH |
| 74 | Benzene ring | Benzene ring | H | 4-OMe | Me | O | CH |
| 75 | Benzene ring | Benzene ring | H | 4-OMe | Et | O | CH |

### <Test Example 1>

The EC₅₀ value of each of the compounds shown in Table 13 with respect to the type 2 ryanodine receptor activity inhibitory effect, was measured in accordance with the following procedure.

### (1) Construction of Expression Plasmid

cDNA of RyR2 was cloned from the heart muscle of a mouse by a PCR method and introduced into an expression vector (pcDNA5/FRT/TO) for Flp-In T-REX having hygromycin resistance. The vector is a tetracycline-inducible expression (Tet-On) vector and induces expression of RyR in the presence of doxycycline.

Also, R-CEPIA1er described in Nat. Commun., 2014,5: 4153 was introduced into a neomycin resistant expression vector, pCMV/myc/ER. This vector constantly expresses R-CEPIA1er.

### (2) Preparation of HEK293 Cell

As HEK293 cells, Flp-In T-REX 293 cells corresponding to the Tet-On inducible system were prepared. The cells were cultured in a CO₂ incubator at 37°C.

### (3) Introduction of R-CEPIA1er and Pathogenic Mutant-Type RyR Gene into HEK293 Cell

A pathogenic mutant-type RyR gene was introduced into Flp-In T-REX 293 cells by a lipofection method. The cells were cultured in a medium containing hygromycin for 10 to 14 days to establish a pathogenic mutant RyR stably expressing strain. Into the pathogenic mutant RyR stably expressing strain, an R-CEPIA1er expression vector was introduced by a lipofection method. The strain was cultured in a medium containing G418 to establish a stable dual expression strain expressing a pathogenic mutant-type RyR gene and R-CEPIA1er.

### (4) Introduction of R-CEPIA1er and Wild-Type RyR Gene into HEK293 Cell

A wild-type RyR gene was introduced into Flp-In T-REX 293 cells by a lipofection method. The cells were cultured in a medium containing hygromycin for 10 to 14 days to establish a RyR stably expressing strain. Into the RyR stably expressing strain, an R-CEPIA1er expression vector was introduced by a lipofection method. The strain was cultured in a medium containing G418 to establish a stable dual expression strain expressing a wild-type RyR gene and R-CEPIA1er.

### (5) Measurement of Ca²⁺ Concentration in Fluorescent Endoplasmic Reticulum

The cultured cells expressing a pathogenic mutant RyR and R-CEPIA1er and the cultured cells expressing wild type RyR and R-CEPIA1er were separately cultured in plastic dishes at 37°C for 24 hours. Then, the medium was exchanged with a medium containing doxycycline and the cells were further cultured for 24 hours. Fluorescence measurement was carried out by use of FlexStation. R-CEPIA1er was excited at 560 nm to acquire fluorescence at 610 nm. Fluorescence was acquired every 10 seconds for 300 seconds. The compounds shown in Table 13 each were added 60 seconds after initiation, and Ca²⁺ concentration in the fluorescent endoplasmic reticulum was measured.

### (6) Evaluation Results of Type 2 Ryanodine Receptor Activity Inhibitory Effect

Inhibitory effects of the compounds on type 2 ryanodine receptor activity shown in Table 13 each were measured in the range of a final concentration of 10⁻⁹ to 10⁻⁴ M by the methods above. Based on the measurement results, a dose-response curve approximating the Hill equation was obtained by the least squares method. In this manner, EC₅₀ values were calculated. The results are shown in Table 13.

Note that, the presence or absence of the effects of compounds 48 to 53, 55 to 58 and 67 to 70 on RyR1 was checked by use of a RyR1 R2163C pathogenic mutation. As a result, an inhibitory effect on RyR1 activity was not confirmed. The presence or absence of effects of compounds 48 to 51, 56, 57 and 67 to 70 on RyR3 was checked. As a result, an inhibitory effect on RyR3 activity was not confirmed.

**[Table 13]**

| Compound No. | EC₅₀ (*µ*M) |
|---|---|
| 61 | 44.2 |
| 66 | 39.5 |
| 40 | 8.31 |
| 58 | 5.46 |
| 45 | 3.37 |
| 38 | 2.77 |
| 62 | 2.68 |
| 59 | 1.36 |
| 47 | 1.25 |
| 63 | 1.05 |
| 60 | 0.768 |
| 35 | 0.676 |
| 37 | 0.606 |
| 41 | 0.154 |
| 46 | 0.147 |
| 56 | 0.060 |
| 53 | 0.051 |
| 51 | 0.049 |
| 54 | 0.048 |
| 49 | 0.044 |
| 48 | 0.043 |
| 67 | 0.041 |
| 69 | 0.039 |
| 50 | 0.036 |
| 70 | 0.033 |
| 68 | 0.031 |
| 57 | 0.015 |

### <Test Example 2>

### (1) Creation of RyR2 Mutation-Harboring Mouse

In Riken BRC, male mice administered with N-ethyl-N-nitrosourea (ENU) were crossed with normal wild type females to obtain child mice (First generation: G1). These G1 mice were subjected to systematic phenotypic screening. Mice exhibiting a heart disease phenotype were analyzed. As a result, a mouse having RyR2 I4093V mutation (RyR2 c.12277A > G (NM_023868.2) p.I4093V (NP_076357.2)) was found. The mouse was backcrossed with a normal wild type mouse (8 generations or more). As a result, mice conceivably having a mutation of RyR2 I4093V alone were obtained.

### (2) Preparation of Solution Containing Compound 57

Compound 57 shown in Table 10 was dissolved in saline (0.9% aqueous NaCl solution) to prepare a 0.03-0.1 mg/mL solution containing compound 57.

### (3) Measurement of Electrocardiogram of RyR2 Mutation-Harboring Mouse and Administration of Drug

The aforementioned mutant mice of 4-5 months old were subjected to anesthesia induction with 2% isoflurane. While maintaining the anesthetic state with 1% isoflurane, electrodes for electrocardiogram were attached to four limbs and electrocardiographical monitoring was continuously carried out. Before drug administration, electrocardiographical monitoring was carried out for 10 minutes. Then, the solution containing compound 57 prepared above was intraperitoneally injected so as to have a concentration of 0.3 mg/kg or 1 mg/kg. The electrocardiogram was recorded for further 15 minutes.

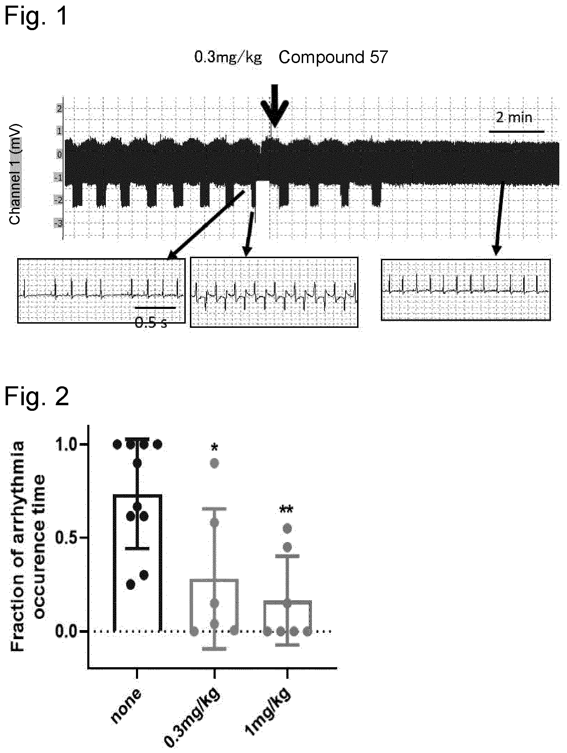

The results of a 0.3 mg/kg intraperitoneal injection case are shown in Figure 1. As shown in Figure 1, bidirectional ventricular tachycardia periodically occurred before administration of compound 57. However, ECG went back to normal by intraperitoneal administration of compound 57 (0.3 mg/kg).

### (4) Results and Consideration

The time during which successive bigeminal pulse, trigeminal pulse or ventricular tachycardia (regarded as arrhythmia) occurred during 5-15 minutes after administration of compound 57, were summed up per individual and the ratio of time when arrhythmia occurs was obtained.

The results are shown in Figure 2. As shown in Figure 2, it is found that in a case where compound 57 is administered in a dose of 0.3 mg/kg or 1 mg/kg, occurrence of arrhythmia is low.

## Claims

1. A type 2 ryanodine receptor activity inhibitor comprising a compound represented by the following formula (α1) or a salt thereof, or a solvate of the compound or a salt thereof, 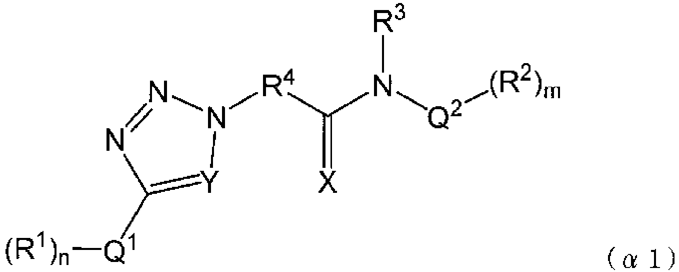 wherein
ring Q¹ and ring Q² each independently represent a condensed ring of a monocyclic heterocycle or monocyclic carbocyclic ring and a benzene ring, or a benzene ring,
R¹ and R² each independently represent a substituted or unsubstituted hydrocarbon group having 1 to 8 carbon atoms, a substituted or unsubstituted alkoxy group having 1 to 8 carbon atoms, a substituted or unsubstituted alkoxycarbonyl group having 2 to 8 carbon atoms, a substituted or unsubstituted alkanoyl group having 2 to 8 carbon atoms, a substituted or unsubstituted alkanoyloxy group having 2 to 8 carbon atoms, a hydroxy group, a cyano group, or a halogen atom,
R³ represents a hydrogen atom or a substituted or unsubstituted hydrocarbon group having 1 to 8 carbon atoms,
R⁴ represents a divalent hydrocarbon group having 1 to 8 carbon atoms,
X represents an oxygen atom or a sulfur atom,
Y represents a methine group or a nitrogen atom, and
n and m each independently represent an integer of 0 to 5;
with the proviso that, if n is an integer of 2 to 5, n number of R¹ may be the same or different and two R¹ may join together to form a ring; and if m is an integer of 2 to 5, m number of R² may be the same or different and two R² may join together to form a ring.

2. The type 2 ryanodine receptor activity inhibitor according to claim 1, wherein ring Q¹ and ring Q² are each independently a condensed ring of a 4 to 8 membered oxygen-containing monocyclic heterocycle and a benzene ring, or a benzene ring.

3. The type 2 ryanodine receptor activity inhibitor according to claim 1 or 2, wherein R¹ and R² are each independently a substituted or unsubstituted alkyl group having 1 to 8 carbon atoms, a substituted or unsubstituted alkenyl group having 2 to 8 carbon atoms, a substituted or unsubstituted alkoxy group having 1 to 8 carbon atoms, a substituted or unsubstituted alkoxycarbonyl group having 2 to 8 carbon atoms, a hydroxy group, a cyano group or a halogen atom.

4. The type 2 ryanodine receptor activity inhibitor according to any one of claims 1 to 3, wherein R⁴ is an alkanediyl group having 1 to 8 carbon atoms or an alkenediyl group having 2 to 8 carbon atoms.

5. The type 2 ryanodine receptor activity inhibitor according to any one of claims 1 to 4, wherein R³ is a hydrogen atom, a substituted or unsubstituted alkyl group having 1 to 8 carbon atoms or a substituted or unsubstituted alkenyl group having 2 to 8 carbon atoms.

6. The type 2 ryanodine receptor activity inhibitor according to any one of claims 1 to 5, wherein R³ is a methyl group.

7. The type 2 ryanodine receptor activity inhibitor according to any one of claims 1 to 6, wherein n is an integer of 1 to 5.

8. The type 2 ryanodine receptor activity inhibitor according to any one of claims 1 to 7, wherein m is an integer of 1 to 5.

9. A medicament for preventing or treating a disease associated with abnormally elevated type 2 ryanodine receptor activity, comprising a compound represented by the following formula (α1) or a salt thereof, or a solvate of the compound or a salt thereof, 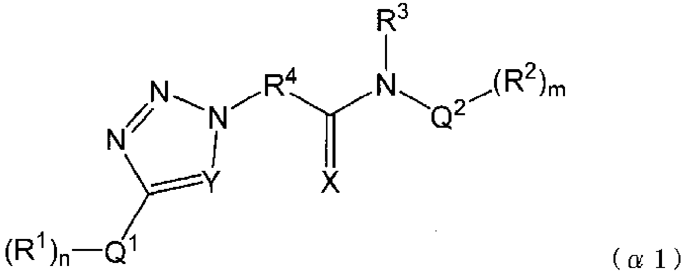 wherein
ring Q¹ and ring Q² each independently represent a condensed ring of a monocyclic heterocycle or monocyclic carbocyclic ring and a benzene ring, or a benzene ring,
R¹ and R² each independently represent a substituted or unsubstituted hydrocarbon group having 1 to 8 carbon atoms, a substituted or unsubstituted alkoxy group having 1 to 8 carbon atoms, a substituted or unsubstituted alkoxycarbonyl group having 2 to 8 carbon atoms, a substituted or unsubstituted alkanoyl group having 2 to 8 carbon atoms, a substituted or unsubstituted alkanoyloxy group having 2 to 8 carbon atoms, a hydroxy group, a cyano group or a halogen atom,
R³ represents a hydrogen atom or a substituted or unsubstituted hydrocarbon group having 1 to 8 carbon atoms,
R⁴ represents a divalent hydrocarbon group having 1 to 8 carbon atoms,
X represents an oxygen atom or a sulfur atom,
Y represents a methine group or a nitrogen atom, and
n and m each independently represent an integer of 0 to 5;
with the proviso that, if n is an integer of 2 to 5, n number of R¹ may be the same or different and two R¹ may join together to form a ring; and if m is an integer of 2 to 5, m number of R² may be the same or different and two R² may join together to form a ring.

10. The medicament according to claim 9, wherein the disease associated with abnormally elevated type 2 ryanodine receptor activity is a disease selected from the group consisting of catecholaminergic polymorphic ventricular tachycardia, idiopathic ventricular fibrillation, arrhythmogenic right ventricular cardiomyopathy, left ventricular noncompaction, epilepsy, mental retardation, chronic heart failure and Alzheimer's disease.

11. A compound represented by the following formula (α2) or a salt thereof, or a solvate of the compound or a salt thereof, 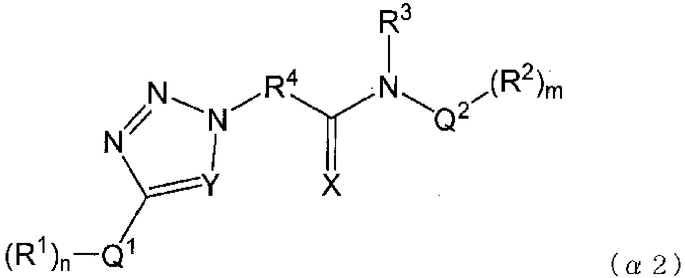 wherein
ring Q¹ and ring Q² each independently represent a condensed ring of a monocyclic heterocycle or monocyclic carbocyclic ring and a benzene ring, or a benzene ring,
R¹ and R² each independently represent a substituted or unsubstituted hydrocarbon group having 1 to 8 carbon atoms, a substituted or unsubstituted alkoxy group having 1 to 8 carbon atoms, a substituted or unsubstituted alkoxycarbonyl group having 2 to 8 carbon atoms, a substituted or unsubstituted alkanoyl group having 2 to 8 carbon atoms, a substituted or unsubstituted alkanoyloxy group having 2 to 8 carbon atoms, a hydroxy group, a cyano group or a halogen atom,
R³ represents a hydrogen atom or a substituted or unsubstituted hydrocarbon group having 1 to 8 carbon atoms,
R⁴ represents a divalent hydrocarbon group having 1 to 8 carbon atoms,
X represents an oxygen atom or a sulfur atom,
Y represents a methine group or a nitrogen atom,
n and m each independently represent an integer of 1 to 5;
with the proviso that, if n is an integer of 2 to 5, n number of R¹ may be the same or different and two R¹ may join together to form a ring; and if m is an integer of 2 to 5, m number of R² may be the same or different and two R² may join together to form a ring.

12. A compound represented by any one of the following formulas (α3) to (α12) or a salt thereof, or a solvate of the compound or a salt thereof,

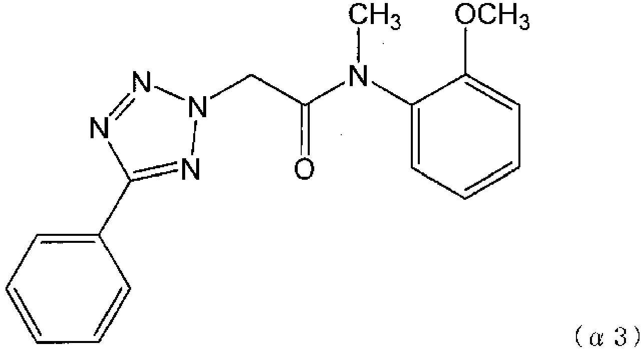

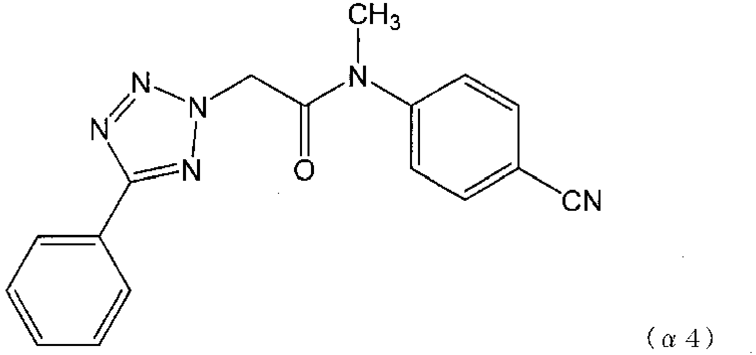

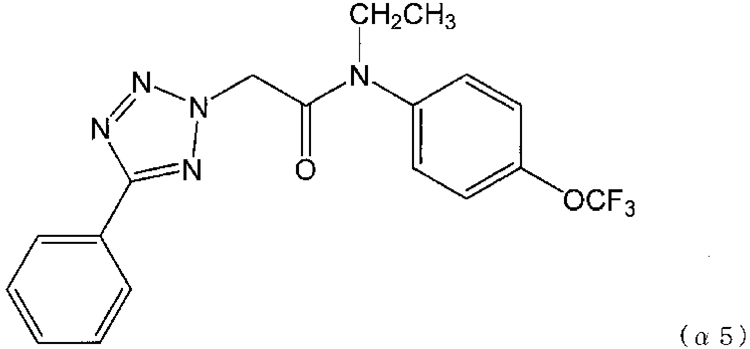

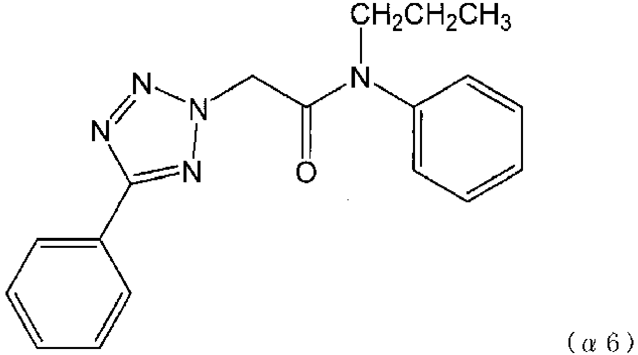

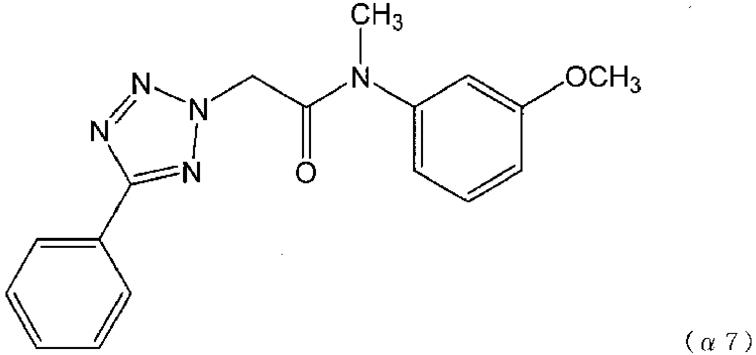

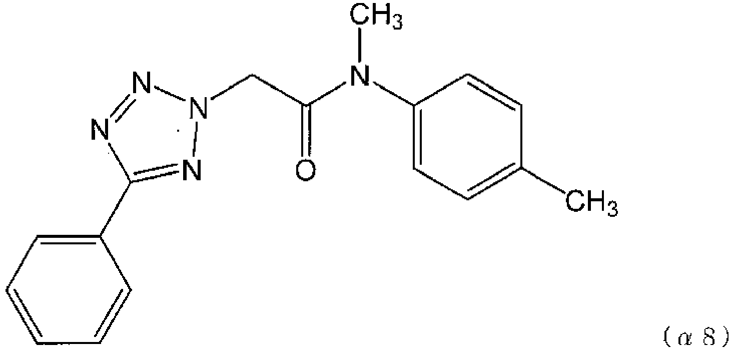

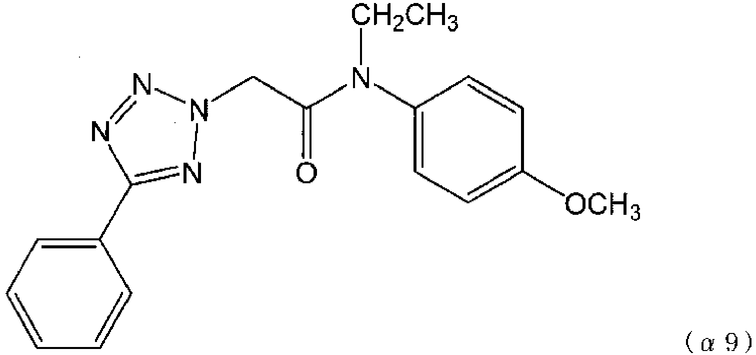

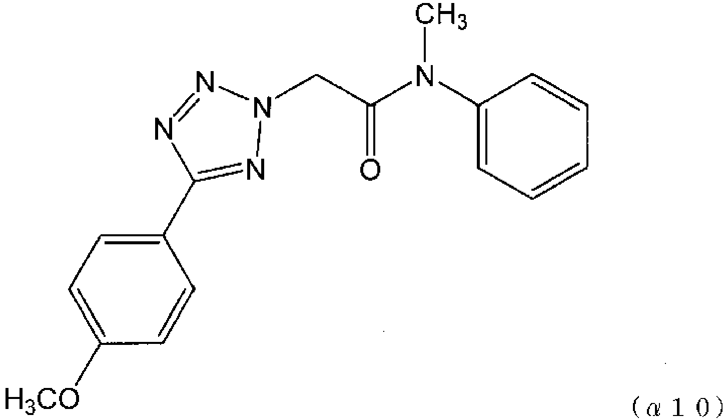

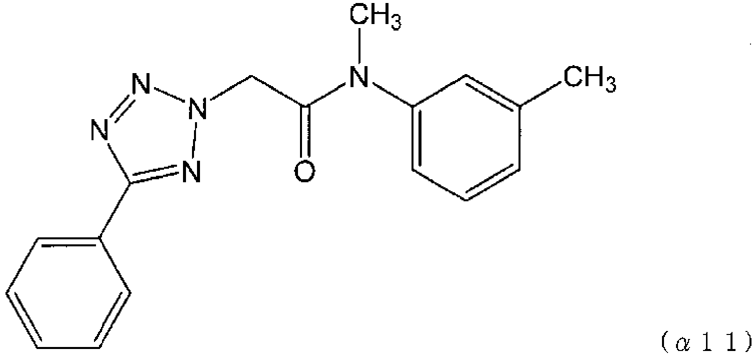

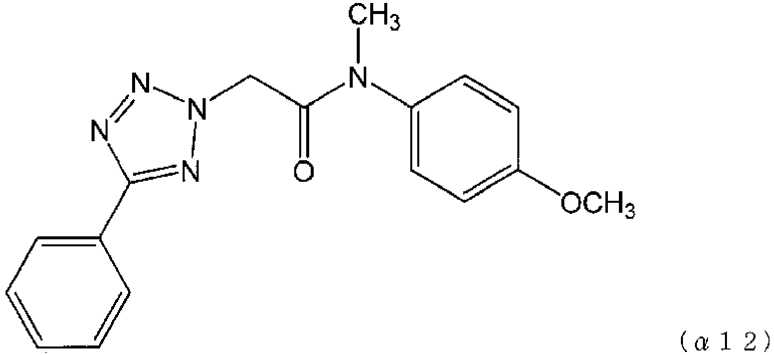

13. Use of a compound represented by the following formula (α1) or a salt thereof, or a solvate of the compound or a salt thereof, for producing a type 2 ryanodine receptor activity inhibitor, 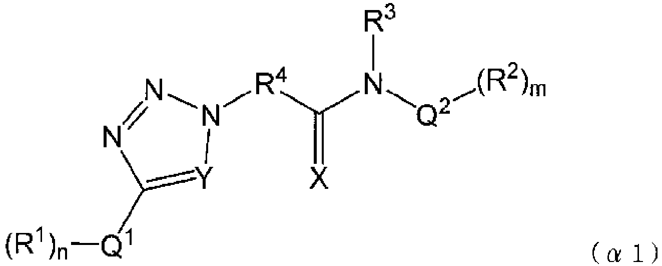 wherein
ring Q¹ and ring Q² each independently represent a condensed ring of a monocyclic heterocycle or monocyclic carbocyclic ring and a benzene ring, or a benzene ring,
R¹ and R² each independently represent a substituted or unsubstituted hydrocarbon group having 1 to 8 carbon atoms, a substituted or unsubstituted alkoxy group having 1 to 8 carbon atoms, a substituted or unsubstituted alkoxycarbonyl group having 2 to 8 carbon atoms, a substituted or unsubstituted alkanoyl group having 2 to 8 carbon atoms, a substituted or unsubstituted alkanoyloxy group having 2 to 8 carbon atoms, a hydroxy group, a cyano group or a halogen atom,
R³ represents a hydrogen atom or a substituted or unsubstituted hydrocarbon group having 1 to 8 carbon atoms,
R⁴ represents a divalent hydrocarbon group having 1 to 8 carbon atoms,
X represents an oxygen atom or a sulfur atom,
Y represents a methine group or a nitrogen atom, and
n and m each independently represent an integer of 0 to 5;
with the proviso that, if n is an integer of 2 to 5, n number of R¹ may be the same or different and two R¹ may join together to form a ring; and if m is an integer of 2 to 5, m number of R² may be the same or different and two R² may join together to form a ring.

14. Use of a compound represented by the following formula (α1) or a salt thereof, or a solvate of the compound or a salt thereof for producing a medicament for preventing or treating a disease associated with abnormally elevated type 2 ryanodine receptor activity, 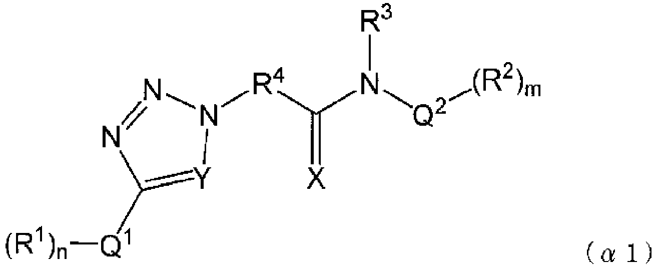 wherein
ring Q¹ and ring Q² each independently represent a condensed ring of a monocyclic heterocycle or monocyclic carbocyclic ring and a benzene ring, or a benzene ring,
R¹ and R² each independently represent a substituted or unsubstituted hydrocarbon group having 1 to 8 carbon atoms, a substituted or unsubstituted alkoxy group having 1 to 8 carbon atoms, a substituted or unsubstituted alkoxycarbonyl group having 2 to 8 carbon atoms, a substituted or unsubstituted alkanoyl group having 2 to 8 carbon atoms, a substituted or unsubstituted alkanoyloxy group having 2 to 8 carbon atoms, a hydroxy group, a cyano group or a halogen atom,
R³ represents a hydrogen atom or a substituted or unsubstituted hydrocarbon group having 1 to 8 carbon atoms,
R⁴ represents a divalent hydrocarbon group having 1 to 8 carbon atoms,
X represents an oxygen atom or a sulfur atom,
Y represents a methine group or a nitrogen atom, and
n and m each independently represent an integer of 0 to 5;
with the proviso that, if n is an integer of 2 to 5, n number of R¹ may be the same or different and two R¹ may join together to form a ring; and if m is an integer of 2 to 5, m number of R² may be the same or different and two R² may join together to form a ring.

15. A compound represented by the following formula (α1) or a salt thereof, or a solvate of the compound or a salt thereof for use in inhibiting a type 2 ryanodine receptor activity, 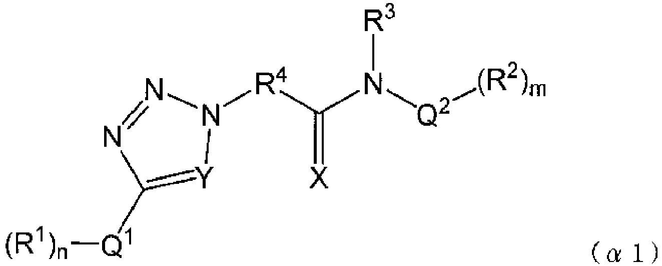 wherein
ring Q¹ and ring Q² each independently represent a condensed ring of a monocyclic heterocycle or monocyclic carbocyclic ring and a benzene ring, or a benzene ring,
R¹ and R² each independently represent a substituted or unsubstituted hydrocarbon group having 1 to 8 carbon atoms, a substituted or unsubstituted alkoxy group having 1 to 8 carbon atoms, a substituted or unsubstituted alkoxycarbonyl group having 2 to 8 carbon atoms, a substituted or unsubstituted alkanoyl group having 2 to 8 carbon atoms, a substituted or unsubstituted alkanoyloxy group having 2 to 8 carbon atoms, a hydroxy group, a cyano group or a halogen atom,
R³ represents a hydrogen atom or a substituted or unsubstituted hydrocarbon group having 1 to 8 carbon atoms,
R⁴ represents a divalent hydrocarbon group having 1 to 8 carbon atoms,
X represents an oxygen atom or a sulfur atom,
Y represents a methine group or a nitrogen atom, and
n and m each independently represent an integer of 0 to 5;
with the proviso that, if n is an integer of 2 to 5, n number of R¹ may be the same or different and two R¹ may join together to form a ring; and if m is an integer of 2 to 5, m number of R² may be the same or different and two R² may join together to form a ring.

16. A compound represented by the following formula (α1) or a salt thereof, or a solvate of the compound or a salt thereof for use in preventing or treating a disease a disease associated with abnormally elevated type 2 ryanodine receptor activity, 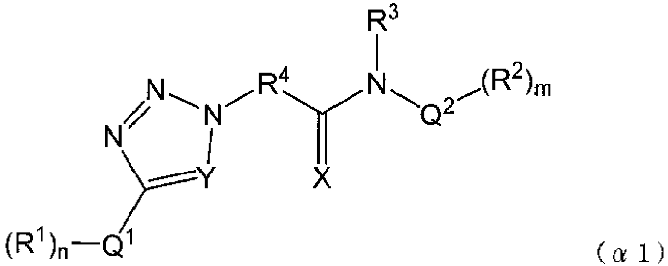 wherein
ring Q¹ and ring Q² each independently represent a condensed ring of a monocyclic heterocycle or monocyclic carbocyclic ring and a benzene ring, or a benzene ring,
R¹ and R² each independently represent a substituted or unsubstituted hydrocarbon group having 1 to 8 carbon atoms, a substituted or unsubstituted alkoxy group having 1 to 8 carbon atoms, a substituted or unsubstituted alkoxycarbonyl group having 2 to 8 carbon atoms, a substituted or unsubstituted alkanoyl group having 2 to 8 carbon atoms, a substituted or unsubstituted alkanoyloxy group having 2 to 8 carbon atoms, a hydroxy group, a cyano group or a halogen atom,
R³ represents a hydrogen atom or a substituted or unsubstituted hydrocarbon group having 1 to 8 carbon atoms,
R⁴ represents a divalent hydrocarbon group having 1 to 8 carbon atoms,
X represents an oxygen atom or a sulfur atom,
Y represents a methine group or a nitrogen atom, and
n and m each independently represent an integer of 0 to 5;
with the proviso that, if n is an integer of 2 to 5, n number of R¹ may be the same or different and two R¹ may join together to form a ring; and if m is an integer of 2 to 5, m number of R² may be the same or different and two R² may join together to form a ring.

17. Use of a compound represented by the following formula (α1) or a salt thereof, or a solvate of the compound or a salt thereof for inhibiting a type 2 ryanodine receptor activity, 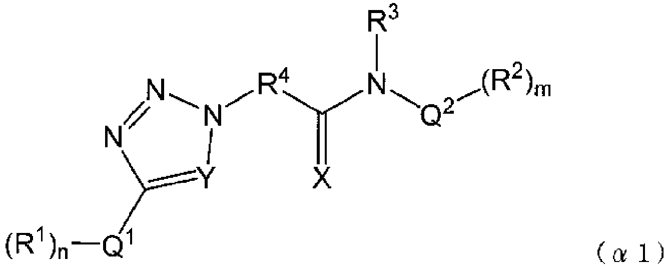 wherein
ring Q¹ and ring Q² each independently represent a condensed ring of a monocyclic heterocycle or monocyclic carbocyclic ring and a benzene ring, or a benzene ring,
R¹ and R² each independently represent a substituted or unsubstituted hydrocarbon group having 1 to 8 carbon atoms, a substituted or unsubstituted alkoxy group having 1 to 8 carbon atoms, a substituted or unsubstituted alkoxycarbonyl group having 2 to 8 carbon atoms, a substituted or unsubstituted alkanoyl group having 2 to 8 carbon atoms, a substituted or unsubstituted alkanoyloxy group having 2 to 8 carbon atoms, a hydroxy group, a cyano group or a halogen atom,
R³ represents a hydrogen atom or a substituted or unsubstituted hydrocarbon group having 1 to 8 carbon atoms,
R⁴ represents a divalent hydrocarbon group having 1 to 8 carbon atoms,
X represents an oxygen atom or a sulfur atom,
Y represents a methine group or a nitrogen atom, and
n and m each independently represent an integer of 0 to 5;
with the proviso that, if n is an integer of 2 to 5, n number of R¹ may be the same or different and two R¹ may join together to form a ring; and if m is an integer of 2 to 5, m number of R² may be the same or different and two R² may join together to form a ring.

18. Use of a compound represented by the following formula (α1) or a salt thereof, or a solvate of the compound or a salt thereof for preventing or treating a disease associated with abnormally elevated type 2 ryanodine receptor activity, 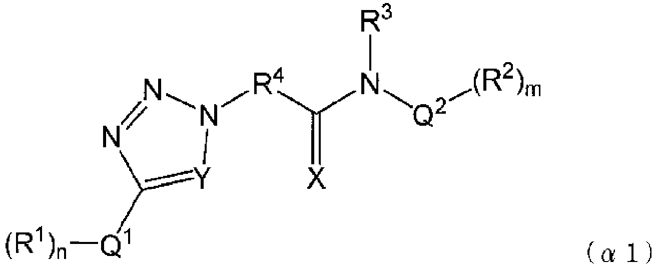 wherein
ring Q¹ and ring Q² each independently represent a condensed ring of a monocyclic heterocycle or monocyclic carbocyclic ring and a benzene ring, or a benzene ring,
R¹ and R² each independently represent a substituted or unsubstituted hydrocarbon group having 1 to 8 carbon atoms, a substituted or unsubstituted alkoxy group having 1 to 8 carbon atoms, a substituted or unsubstituted alkoxycarbonyl group having 2 to 8 carbon atoms, a substituted or unsubstituted alkanoyl group having 2 to 8 carbon atoms, a substituted or unsubstituted alkanoyloxy group having 2 to 8 carbon atoms, a hydroxy group, a cyano group or a halogen atom,
R³ represents a hydrogen atom or a substituted or unsubstituted hydrocarbon group having 1 to 8 carbon atoms,
R⁴ represents a divalent hydrocarbon group having 1 to 8 carbon atoms,
X represents an oxygen atom or a sulfur atom,
Y represents a methine group or a nitrogen atom, and
n and m each independently represent an integer of 0 to 5;
with the proviso that, if n is an integer of 2 to 5, n number of R¹ may be the same or different and two R¹ may join together to form a ring; and if m is an integer of 2 to 5, m number of R² may be the same or different and two R² may join together to form a ring.

19. A method for inhibiting a type 2 ryanodine receptor activity, comprising a step of administering a compound represented by the following formula (α1) or a salt thereof, or a solvate of the compound or a salt thereof, 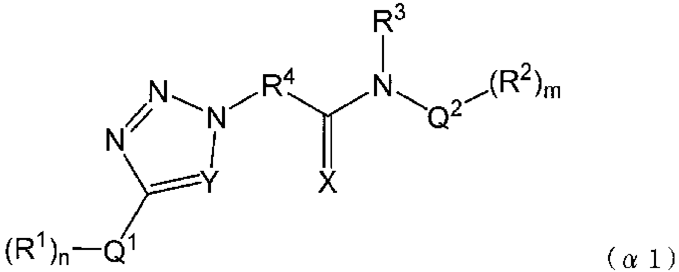 wherein
ring Q¹ and ring Q² each independently represent a condensed ring of a monocyclic heterocycle or monocyclic carbocyclic ring and a benzene ring, or a benzene ring,
R¹ and R² each independently represent a substituted or unsubstituted hydrocarbon group having 1 to 8 carbon atoms, a substituted or unsubstituted alkoxy group having 1 to 8 carbon atoms, a substituted or unsubstituted alkoxycarbonyl group having 2 to 8 carbon atoms, a substituted or unsubstituted alkanoyl group having 2 to 8 carbon atoms, a substituted or unsubstituted alkanoyloxy group having 2 to 8 carbon atoms, a hydroxy group, a cyano group or a halogen atom,
R³ represents a hydrogen atom or a substituted or unsubstituted hydrocarbon group having 1 to 8 carbon atoms,
R⁴ represents a divalent hydrocarbon group having 1 to 8 carbon atoms,
X represents an oxygen atom or a sulfur atom,
Y represents a methine group or a nitrogen atom, and
n and m each independently represent an integer of 0 to 5;
with the proviso that, if n is an integer of 2 to 5, n number of R¹ may be the same or different and two R¹ may join together to form a ring; and if m is an integer of 2 to 5, m number of R² may be the same or different and two R² may join together to form a ring.

20. A method for preventing or treating a disease associated with abnormally elevated type 2 ryanodine receptor activity, comprising a step of administering a compound represented by the following formula (α1) or a salt thereof, or a solvate of the compound or a salt thereof, 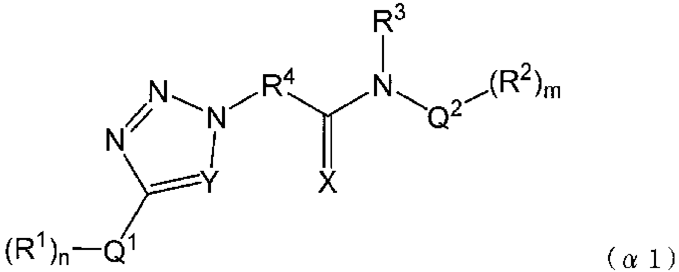 wherein
ring Q¹ and ring Q² each independently represent a condensed ring of a monocyclic heterocycle or monocyclic carbocyclic ring and a benzene ring, or a benzene ring,
R¹ and R² each independently represent a substituted or unsubstituted hydrocarbon group having 1 to 8 carbon atoms, a substituted or unsubstituted alkoxy group having 1 to 8 carbon atoms, a substituted or unsubstituted alkoxycarbonyl group having 2 to 8 carbon atoms, a substituted or unsubstituted alkanoyl group having 2 to 8 carbon atoms, a substituted or unsubstituted alkanoyloxy group having 2 to 8 carbon atoms, a hydroxy group, a cyano group or a halogen atom,
R³ represents a hydrogen atom or a substituted or unsubstituted hydrocarbon group having 1 to 8 carbon atoms,
R⁴ represents a divalent hydrocarbon group having 1 to 8 carbon atoms,
X represents an oxygen atom or a sulfur atom,
Y represents a methine group or a nitrogen atom, and
n and m each independently represent an integer of 0 to 5;
with the proviso that, if n is an integer of 2 to 5, n number of R¹ may be the same or different and two R¹ may join together to form a ring; and if m is an integer of 2 to 5, m number of R² may be the same or different and two R² may join together to form a ring.
